# EUROPEAN PATENT SPECIFICATION

(11) **EP 3 131 546 B1**
(45) Date of publication and mention of the grant of the patent: **16.02.2022**
(21) Application number: 15779860.4
(22) Date of filing: 16.04.2015
(51) Int. Cl.: A61K 9/14, A61K 31/436, A61P 1/02, A61K 9/16, A61K 9/20

(54) **ORAL RAPAMYCIN PREPARATION FOR USE IN TREATING FELINE CHRONIC GINGIVO- STOMATITIS (FCGS)**
ORALE RAPAMYCINFORMULIERUNG ZUR VERWENDUNG BEI DER BEHANDLUNG VON FELINER CHRONISCHER GINGIVO-STOMATITIS (FCGS)
PRÉPARATION ORALE DE RAPAMYCINE POUR UTILISATION DANS LE TRAITEMENT DE LA GINGIVO-STOMATITE CHRONIQUE FÉLINE (FCGS)

(30) Priority: 16.04.2014 US 201461980095 P
(43) Date of publication of application: 22.02.2017
(73) Proprietor: Rapamycin Holdings, Inc., San Antonio, TX 78249 (US)
(72) Inventor: VAUGHN, Dana, M., Seguin, TX 78155 (US)
(74) Representative: Forresters IP LLP
(86) International application number: PCT/US2015/026266
(87) International publication number: WO 2015/161139

(56) References cited:
- WO-A1-2006/066063
- WO-A1-2014/144346
- WO-A1-2014/144405
- WO-A2-03/106382
- WO-A2-2006/110862
- WO-A2-2010/056754
- US-A- 5 952 373
- US-A1- 2008 107 749
- US-A1- 2010 105 637
- US-A1- 2011 311 596
- US-A1- 2012 064 143
- US-A1- 2012 276 169
- KENNETH F. LYON: "Gingivostomatitis", VETERINARY CLINICS OF NORTH AMERICA: SMALL ANIMAL PRACTICE., vol. 35, no. 4, 1 July 2005 (2005-07-01), pages 891-911, XP055393931, US ISSN: 0195-5616, DOI: 10.1016/j.cvsm.2005.02.001
- Fraser A Hale ET AL: "Hale Veterinary Clinic Long Distance: 1", , 1 December 2010 (2010-12-01), XP055426108, Retrieved from the Internet: URL:http://toothvet.ca/PDFfiles/fcgs.pdf [retrieved on 2017-11-16]

## Description

### BACKGROUND

### 1. Field of the Invention

The present disclosure relates generally to feline healthcare and particularly to prevention, treatment and management of stomatitis, most particularly feline chronic gingivo-stomatitis (FCGS). More particularly, aspects of the disclosure relate to manufacture and use of orally administered pharmaceutical preparations for prevention, treatment and management of feline chronic gingivo-stomatitis (FCGS).

### 2. Description of Related Art

For these purposes, "stomatitis" refers generically to any oropharyngeal inflammation and related processes of the mucous membranes in or around the mouth and oropharyngeal tissues of a subject. When stomatitis involves inflammation of the gums (i.e., gingiva), it can be generally referred to as gingivo-stomatitis, irrespective of whether mucous membranes other than the gums are actually inflamed. To make more specific reference to inflammation of the gums, however, it is sometimes referred to as just "gingivitis." Because of the critical association between the gums and dental hygiene, for which numerous consumer products are targeted, at least for human healthcare markets, most consumers are somewhat familiar with the "gingivitis" term, although any form of gingivo-stomatitis can lead to serious complications.

Although the risks and challenges of gingivo-stomatitis can be serious for any species, gingivo-stomatitis can be especially devastating in feline populations. The "chronic" aspects of FCGS applies if a cat's natural healing mechanisms are unable to reverse the condition which is often presumed if a cat's gingiva-stomatitis does not resolve within thirty days of onset. Once the condition progresses to a chronic condition, then, unfortunately, the prognosis for cats with FCGS tends to be life changing. Many veterinarians routinely consider full mouth dental extraction and root removal as the most practical treatment if not an essential part of managing FCGS.

Though domestic cats can often live long lives despite dental extraction, the resulting lifestyle limitations can be life altering. A cat's teeth are not only important for eating, but also for self-defense. Moreover, their teeth often play an important role in their social relationships, especially with male cats, as a male cat will typically use its teeth to hold onto the neck of a female cat during copulation. Other challenges can be understood from published scientific literature on the subject, such as the December 2010 article entitled "The Disease Formerly Known as Lymphocytic/Plasmacytic Gingivo-Stomatitis," which can be accessed on the Internet through the website located at URL www-toothvet.ca*.*

Despite the well-known and long-felt needs for developing a pharmaceutical intervention for FCGS, no pharmaceutical interventions have demonstrated sufficient efficacy to be recognized as a routine intervention option for veterinarians. Moreover, even if one were contemplating active ingredients for preventing, treating or managing FCGS without the benefit of the present invention and impermissible hindsight, the teachings of the present invention would not be realistic candidates. Not only are Applicant's present teachings not known to be efficacious in the art of treating FCGS, but such teachings would likely be summarily dismissed from consideration even if a related thought ever arose. Such summarial dismissal would seem inevitable, largely because, if for no other reason, many have assumed that some aspects of the present invention present a material risk of ulcerating oropharyngeal tissues.

Irrespective of the state of art in the present field, for some applications far outside the scope and field of the present invention, rapamycin (also known as sirolimus) is a well-known pharmaceutical agent. Most notably, rapamycin has long been successfully used to minimize organ transplant rejection in humans, while seemingly countless other potential applications have also been postulated from time to time.

Rapamycin and its numerous analogs and derivatives (collectively known as "rapalogs") famously act to inhibit its namesake metabolic pathway in mammals -- the mammalian target of rapamycin ("mTOR"). The critical metabolic roles of the mTOR pathway have long led to broad speculation about possible medical uses for rapamycin in addition to its well-known efficacy in reducing human organ transplant rejection. However, despite the success with prevention of transplant rejection, and despite the many long-felt needs and corresponding tremendous efforts in developing rapamycins for other indications, effective use of rapamycin for treating or preventing other disorders has not been widely successful and has been very limited at best.

Particular formulations (US Patent Application Serial No. 13/128,800, filed November 11, 2009, published under Publication No. 2012/0064143, the "2008 Discoveries") provided particles or "cores" containing the active rapamycin ingredient, and those cores were microencapsulated within a protective polymer matrix, for oral administration of the rapamycin. The rapamycin cores were preferably microencapsulated using a spinning disk atomization coating process with a protective polymer matrix known under the "EUDRAGIT^{®} S 100" name. The EUDRAGIT^{®} S 100 polymer matrix principally consists of a particular methacrylate polymer that is generally stable at pH levels below 7 and was thought to protect rapamycin from degrading in acidic conditions of the stomach. Then, once the microencapsulated rapamycin entered basic conditions (i.e., pH greater than 7) within the intestines, the protective matrix would be able to dissolve and, theoretically, undegraded rapamycin would then be bioavailable for absorbtion through the intestinal walls of the subject. However, despite tremendous hope for broad efficacy of orally administered use of such microencapsulated rapamycin preparations, and despite widespread national and international attention to the 2008 Discoveries, commercial acceptance of the 2008 Discoveries has been minimal if not non-existent, as formidable challenges have remained.

Still, though, referring again to the field of the present invention, there remain long-felt unresolved needs in improving feline healthcare by providing an efficacious pharmaceutical preparation for treating and otherwise managing FCGS. Many other secondary needs and objectives will be understood by those of skill in the art.

US 2008/107749 discloses polymeric nanoparticles with a hydrophobic core and a hydrophilic shell formed from: 1) N-isopropyl acrylamide (NIPAAM), at a molar ratio of about 50% to about 90%, and preferably 60% for specific delivery routes such as oral or parenteral; either water-soluble vinyl derivatives like vinylpyrolidone (VP) or vinyl acetate (VA), or water insoluble vinyl derivatives like methyl methacrylate (MMA) or styrene (ST), at a molar ratio of about 10% to about 30%; and acrylic acid (AA), at a molar ratio of about 10% to about 30%.

WO 2006/110862 discloses that hydrophobic drags become more practical for treatments by being encapsulated in micelle compositions for increasing solubility. The micelle compositions disclosed may include an excipient tocopherol and/or prodrug formulations of the drug. It is also disclosed that micelles extend the time period the drug remains in the micelles to improve drug circulation time and thereby drug delivery. The disclosed hydrophobic drugs for micelle encapsulation may include rapamycin, geldanamycin, and paclitaxel.

WO 2010/056754 discloses microcapsules that include an inhibitor of the mammalian target of rapamycin (mTOR) within the microcapsules, and pharmaceutical compositions and kits that include the microcapsules. Also disclosed are methods for treating or preventing an age- related disease, condition, or disorder in a subject that involve administering to a subject a pharmaceutically effective amount of microcapsules that includes an inhibitor of mTOR within the microcapsules. KENNETH F. LYON, "Gingivostomatitis", VETERINARY CLINICS OF NORTH AMERICA: SMALL ANIMAL PRACTICE., US, (20050701), vol. 35, no. 4, ISSN 0195-5616, pages 891 - 911, XP055393931 discloses therapeutics used in human oral mucosal diseases including inhibitors of cytokine and growth factor action (i.e. tacrolimus, sirolimus, leflunomide). Also disclosed are various treatments for gingivostomatitis in felines.

WO 2014/144346 discloses methods and compositions for preventing cerebrovascular function dysfunction in a patient who has been identified as an ApoE4 carrier. The disclosed methods and compositions include rapamycin, a rapamycin analog, or another such inhibitor of the target of rapamycin (TOR).

WO 2014/144405 discloses methods and compositions for the prevention or inhibition of the growth of endocrine-related adenomas, neoplasia, or dysplasia in a patient who has been identified as being at risk for developing an endocrine tumor or endocrine cancer. The disclosed methods and compositions include rapamycin, a rapamycin analog, or another such inhibitor of the target of rapamycin (TOR).

WO 2006/066063 discloses nanoparticulate tacrolimus compositions. The disclosed composition comprises tacrolimus particles having an effective average particle size of less than about 2000 nm and at least one surface stabilizer.

### SUMMARY OF THE INVENTION

The invention is defined by the claims.

While the present disclosure is multifaceted and can be embodied in many other forms, some aspects of the disclosure are embodied as methods or preparations for treating feline chronic gingivo-stomatitis (FCGS) and related conditions. Some disclosures also relate to preventing or managing FCGS and related conditions. Some teachings are also embodied as methods of administering improved forms of microencapsulated rapamycins and in methods for reliably producing and administering such improved forms relative to subjects. Related disclosures also represent pharmaceutical preparations that would be suitable for such uses, as well as methods for making such pharmaceutical preparations. Even in the event disclosures are not well described in those terms, the reader of skill in feline healthcare arts should still understand the characteristics of these disclosures based on the modes of disclosure and the various disclosures that are described hereafter.

Even though the disclosure relates primarily to feline healthcare and to prevention, treatment and management of FCGS and related conditions, many of the methods and preparations of the present disclosure involve improved forms of microencapsulated rapamycins, most preferably in the form of nanoparticles containing mTOR inhibitors stabilized and protected within a methyl methacrylate polymer matrix. Characteristics of such preparations are particularly beneficial for improving the stability of the subject mTOR inhibitor and for improving its bioavailability despite oral administration. The resulting preparations are not only more durable and stable, but are also more bioavailable and efficacious for treatment, prevention and management of feline chronic gingivo-stomatitis (FCGS). Such preparations also improve stability and bioavailability of rapamycin or other mTOR inhibitors for use in preventing, treating or managing autoimmune mucosal disorders and their precursors, concomitants and sequelae in humans and other animal subjects.

In some disclosed methods of administering a preparation and some disclosed methods of treatment, mTOR inhibiting preparations are administered in any desired manner. However, preferred disclosures involve making or obtaining preparations of rapamycin or an analog of rapamycin that is orally administered to the subject in multiple dosings over a multi-week period.

The compositions administered to the subjects preferably comprise rapamycin, or an analog of rapamycin or an alternate mTOR inhibitor. The more preferred forms of such compositions include a nanoparticle construct combined with a carrier material, preferably an enteric composition, for purposes of minimizing degradation of the composition until it passes the pylorus to the intestines of the subject. Compositions comprising rapamycin or an analog of rapamycin may also include a hydrophilic, swellable, hydrogel-forming material. Such compositions may be encased in a coating that includes a water insoluble polymer and a hydrophilic water permeable agent. In some embodiments, the water insoluble polymer is a methyl methacrylate-methacrylic acid copolymer. Compositions comprising rapamycin or an analog of rapamycin may further include a thermoplastic polymer for purposes of gradual or controlled release of the rapamycin or an analog of rapamycin. Examples of the thermoplastic polymer include EUDRAGIT^{®} Acrylic Drug Delivery Polymers (Evonik Industries AG, Germany).

In some preferred preparations that are used for preventing, treating or managing the targeted maladies, rapamycin particles or particles of rapamycin analogs or other mTOR inhibitors or analogs thereof, are encapsulated or coated. In other preferred preparations, a more complex composition that includes the rapamycin or other mTOR inhibitor or analog thereof is encapsulated or coated. For reference purposes in these descriptions, "microencapsulation" (and its grammatical variations) should be interpreted to refer to protection of microparticle or nanoparticle forms of rapamycins (preferably in the nanoparticle forms according to the descriptions herein) by combining such particles with an enteric coating material or the like that is formulated to resist degradation in acidic conditions. Further, the designations "microencapsulated rapamycin" and "enteric-coated rapamycin" are used interchangeably to refer generically to each and every variation of microencapsulated rapamycins, especially to those variations that are described or particularly suggested in these descriptions, and equivalents thereof. Exceptions in particular contexts should be understood, nonetheless, to the extent that the context makes more specific or contrary clarifications for that context. In some embodiments, the encapsulant or coating used for and incorporated in enteric-coated rapamycin preparations may be an enteric coating. In another aspect of these descriptions, general references to "prevention and treatment" (or the like) of a malady should be interpreted to include reference not only to prevention and treatment of the actual malady, but also to delay or reduction in the progression of that malady as well as prevention and treatment of its precursors, concomitants and sequelae.

In many embodiments involving enteric-coated rapamycin preparations, the rapamycins or other mTOR inhibitors, or related compositions that include the enteric-coated rapamycin preparations, are provided in the form of nanoparticles that include the rapamycin or other mTOR inhibitor within a pharmaceutically active core. In such cases, the designation "nanoRapa" is generically used for reference purposes in these descriptions. While the form of rapamycins used for a described embodiment may preferably include (but not be limited to) an encapsulated form of a nanoRapa preparation, such encapsulated forms of nanoparticles are occasionally designated as "enteric-coated rapamycin nanoparticles" or more simply as "eRapaNP2g" or "e-nanoRapa."

After preparing or otherwise obtaining nanoRapa preparations through any of various approaches that may be understood and/or described herein, the nanoRapa preparation may then be coated with an enteric coating to provide an enteric-coated rapamycin preparation formed from nanoRapa particles. For reference purposes in these descriptions, the designation "e-nanoRapa" is generically used to refer to each and every enteric-coated rapamycin variation formed from nanoRapa particles.

Many other objects, features and advantages of the present invention will become apparent to those of ordinary skill in the art, particularly after a thorough review of the public literature in the field, and all the more from the following detailed descriptions and accompanying illustrations and claims. It should be understood, however, that the detailed description and the specific examples, while indicating preferred embodiments of the invention, are given by way of illustration only, since various changes and modifications within the scope of the claimed invention will become apparent to those skilled in the art from these detailed descriptions.

### BRIEF DESCRIPTION OF THE DRAWINGS

The accompanying drawings form part of the present specification and are included to further demonstrate and illustrate certain aspects of the present invention. The invention may be better understood by reference to one or more of these drawings in combination with the detailed description presented herein.
**Fig. 1** is a flowchart illustration of a basic approach for preferred methods for using teachings of the present disclosure in response to a case of suspected FCGS, which also provides an illustrative reference for other methods and embodiments described in more detail in the text.
**Fig. 2** is a graphic illustration of microscopic aspects of a preferred process for producing a dispersion of preferred forms of rapamycin nanoparticles according to the teachings of the present disclosure.
**Fig. 3** is a graphic illustration of two basic steps in a preferred process for producing a dispersion of preferred forms of rapamycin nanoparticles according to the teachings of the present disclosure.
**Fig. 4** provides a photograph of a dispersion of rapamycin nanoparticles produced as a result of Step 2 in the preferred process illustrated in **Fig. 3****,** together with a graph of nanoparticle size distribution for the dispersion shown in the photograph.
**Figs. 5A** and **5B** are flowcharts illustrating detailed steps of a more comprehensive preferred process for producing preferred forms of enteric-coated rapamycin nanoparticles, which includes the process for producing a nanoparticle dispersion as illustrated in **Figs. 2** and **3****,** together with additional steps for microencapsulating the rapamycin nanoparticles.
**Figs. 6A** and **6B** present summary data to illustrate how extended regular use of microencapsulated rapamycin nanoparticles was effective at reducing feline chronic gingivo-stomatitis (FCGS) disease scores in 100% of sixteen feline subjects.
**Figs. 7A** and **7B** present summary data to illustrate how extended regular use of microencapsulated rapamycin nanoparticles administered was effective at reducing FCGS disease scores in 90% of ten feline subjects, even at a lower dose than what was administered in a previous study, the results of the previous study being represented in **Figs. 6A** and **6B****.**

### DETAILED DESCRIPTION OF PREFERRED EMBODIMENTS

The following descriptions are provided to illustrate further detail of preferred embodiments of the invention. It should be appreciated by those of skill in the art that the treatment protocols, formulations and techniques disclosed are thought to represent embodiments that function well in the practice of the invention, and thus can be considered to constitute preferred modes for its practice. However, in light of the present disclosure, those of skill in the art should also appreciate that many changes can be made in any of these embodiments and the way they work while still obtaining equivalent results, without departing from the scope of the claimed invention.

For purposes of these descriptions, a few wording simplifications should be understood as universal, except to the extent otherwise clarified in a particular context either in the specification or in any claims related to these descriptions. The use of the term "or" in the specification is used to mean "and/or" unless explicitly indicated to refer to alternatives only, or unless the alternatives are inherently mutually exclusive. When referencing values, the term "about" is used to indicate an approximate value, generally one that at least includes values within at least a standard deviation of error for any particular embodiments that are disclosed or that are commonly used for determining such value. "A" or "an" may mean one or more, unless clearly indicated otherwise. Such "one or more" meanings are most especially intended when references are made in conjunction with open-ended words such as "having," "comprising" or "including." Likewise, "another" may mean at least a second or more.

### TREATING FCGS & MANAGING STOMATITIS RELATED CONDITIONS

Basic aspects of the present disclosure enable pharmaceutical preparations and associated treatment regimens that are surprisingly successful in treating and otherwise managing stomatitis, most particularly for feline chronic gingivo-stomatitis (FCGS). Although numerous variations are contemplated for applying the disclosure in the treatment of FCGS and in the management of related health conditions, preferred embodiments involve a regimen of orally-administering stable preparations of mTOR inhibitors multiple times per week over a multi-week duration. While further particulars will be understood in the course of these descriptions, we first describe various approaches for producing or otherwise obtaining suitable mTOR inhibitor preparations, many of which are suitable for use in practicing the methods of the present disclosure. Later portions of these descriptions also describe particular examples of how the invention has been and can be used in practice.

More particularly, multi-week regimens of orally administered microencapsulated mTOR inhibiting nanoparticles are surprisingly effective in treating and reducing the severity of FCGS. Although potential dosing variations will also be understood to those of skill in the art, these descriptions will also elaborate on particular examples that are known to deliver preferred mTOR inhibitors in bioavailable amounts that have now been found to be clearly efficacious for reducing the severity of FCGS in most feline subjects. Indeed, some subjects that had quantifiable levels of FCGS upon commencement of a multi-dose treatment regimen appear to be fully cured of FCGS after just two weeks of following a preferred regimen, and it may well be that those subjects were fully cured even prior to completion of the two-week duration. These and other aspects of how to practice the invention will be described with reference to specific examples further in these descriptions.

Despite more rapid results in some subjects, the most reliable regimens of the present invention involve oral administration of stable mTOR inhibiting preparations multiple times per week over durations of four weeks or more. Preferred regimens follow a daily administration of the mTOR inhibiting preparations, while other dosing regimens of every other day or three-times-per-week are also suitable. To achieve optimum pharmacokinetics, it should be understood of course that optimal daily doses contain smaller quantities of the bioavailable mTOR inhibitor than do less frequent doses.

Vice versa, it should also be understood that the amount of bioavailable mTOR inhibitor per dose can be reduced below preferred levels of a daily dose as needed, so long as other accommodations are made to ensure ingestion and metabolic absorption of efficacious amounts of the mTOR inhibitor. As one example of an alternative using smaller amounts of bioavailable mTOR inhibitor per dose, although daily oral administration is thought to be as frequent as would be necessary for achieving efficacious levels in a subject, smaller bioavailable amounts per dose of the mTOR inhibitor can be used as alternatives if for some reason a regimen is desired with multiple oral administrations per day. Those of skill in the art can readily determine suitable dose characteristics for such multiple administrations per day by resolving the equivalent of any of the daily doses described herein.

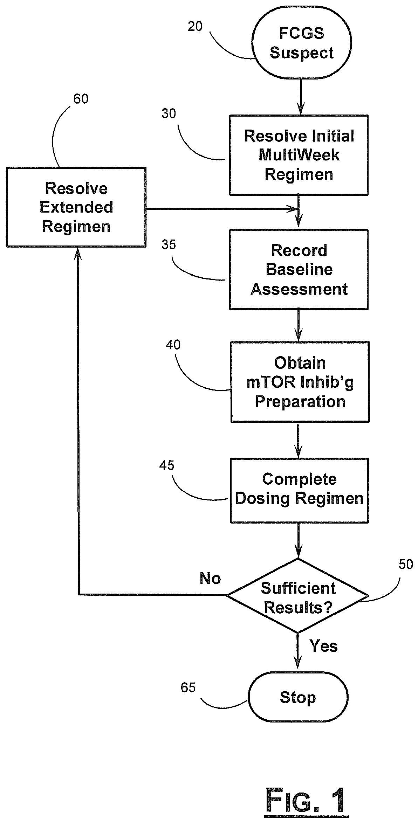

Based in part on the various studies described herein and the surprisingly efficacious results demonstrated by such studies, preferred methods of treating or managing FCGS have been developed. With reference to the flowchart of **Fig. 1****,** there is shown a context for general characteristics of such preferred methods. Preferably, a preferred method is commenced once a veterinarian determines that a feline subject is suspected of having FCGS, as represented by starting point **20** in **Fig. 1****.** In practice, starting point **20** preferably includes a visual assessment of the condition of the subject's oropharyngeal mucous membranes by a properly trained veterinarian or feline healthcare specialist. Though not illustrated in detail in **Fig. 1****,** starting point **20** may also be embodied or practiced in a manner to include conducting more confirmatory tests or the like that reach the level of clearly establishing that the subject has FCGS. Starting point **20** may also be embodied to include various steps to eliminate nutrition, infection or other dental conditions (e.g., dental cleaning to remove plaque that can exacerbate gingival irritation) to the extent such steps can be easily completed without risk to the subject.

Though not separately shown in **Fig. 1****,** preferred methods also include a preparatory regimen prior to administration of the first dose of the mTOR inhibitor. Preferred preparatory regimens include (a) one or more dental cleanings, (b) a period of antibiotic pre-treatment to ensure that the subject's stomatitis cannot be fully addressed without resort to an mTOR-inhibiting regimen, and (c) assessing, photographing and otherwise establishing a baseline for measuring progress during treatment with an mTOR inhibiting regimen. One or more dental cleanings are preferably part of the preparatory regimen in order to minimize plaque and other secondary factors that may irritate the gums or otherwise contribute to or exacerbate FCGS. Further dental cleanings and tissue management measures may also be in order during the course of administering the mTOR inhibitor, according to physician discretion.

With respect to antibiotic pre-treatments, the general purpose for as much is to ensure that the subject's stomatitis cannot be fully addressed by treating an underlying infection, without resort to an mTOR-inhibiting regimen. The preferred duration for such a pre-treatment is two weeks, although other durations will be understood as alternatives for achieving much the same purposes. If the severity of the subjects' stomatitis is not significantly reduced after a reasonable period of antibiotic pre-treatment, then the FCGS is understood to be refractory, and if not resolved after 30 days of alternative treatment attempts, the FCGS is confirmed as chronic.

As illustrated next in the flowchart of **Fig. 1****,** once observations made cause the veterinarian or other caregiver to at least suspect, or even know, that the subject has FCGS at Starting Point **20,** the veterinarian or caregiver then resolves the desired characteristics for an initial multi-week regimen of administering mTOR inhibiting preparations to the subject, which is illustrated as step **30** in the flowchart of **Fig. 1****.** The initial multi-week regimen to be followed is preferably about two weeks or longer in duration, and it preferably involves administration of mTOR inhibiting preparations in doses and at frequencies in accord with other descriptions in this document. Preferably, the resolved regimen administers such preparations in a manner such that said feline subject ingests amounts of a pharmaceutically active mTOR inhibitor compound prepared according to the teachings in other portions of this description, in amounts and frequencies and over durations that are efficacious for reducing the severity of, and preferably to fully cure, FCGS in the feline subject.

After the initial regimen is resolved at Step **30,** the initial multi-week dosing regimen is followed for the initial duration, as represented by Step **45** in **Fig. 1****.** However, *before* commencing that dosing regimen **45,** it is also preferred that the veterinarian staff create an initial baseline record of the subject's FCGS, as illustrated at Step **35.** The Step **35** of recording an initial baseline assessment preferably includes charting the extent of the FCGS, compiling photographs or diagrams of affected tissue, and conducting an assessment of FCGS severity using recognized FCGS severity rating scales, such as the 4-point scale described elsewhere herein.

Particular regimens for administration of the mTOR inhibitor according to the present invention preferably involve oral administration of eRapaNP2g in capsule form, over the duration of the multi-week initial regimen, which is preferably a four- or six- or eight-week duration. Recording the initial baseline assessment at Step **35** preferably serves to later aid in assessing progress, by providing a baseline for comparing to the results of administering the mTOR-inhibiting treatment regimen as

Though less critical, establishing an initial baseline at Step **35** may also include performing biopsies of affected tissue, such as were performed in the course of the studies described elsewhere herein. Such biopsies may be taken by punch biopsy or excision prior to eRapaNP2g administration at an initial oral examination. For comparison and assessment of progress, such biopsies may again be taken following the oral examination at the termination of treatment regimen, which is preferably part of Decision Point **50** in **Fig. 1****.** When an initial baseline assessment **35** involves a biopsy, the site of the biopsy is preferably selected from a region of greatest inflammation that is large enough such that subsequent biopsies can also be performed in the same area for comparison. For best comparisons, a final biopsy will preferably be taken from a region of tissue immediately adjacent to the original biopsy site.

By comparing to the initial baseline record **35** after completion of the initial regimen **45,** the Decision Point **50** that asks whether the results of the initial regimen are sufficient can be more readily and reliably completed. If the results are sufficient, then treatment may be stopped, as represented by Stopping Point **65** in Fig. 1; whereas if the results are considered insufficient by the veterinarian, she or he can then resolve the characteristics of a second multi-week regimen of administering mTOR inhibiting preparations according to the teachings of the present invention, as represented by Step **60** in **Fig. 1****.** The characteristics for an extended regimen resolved at Step **60** can be much the same as the initial regimen, although the dosing amount and/or frequency may be increased in the discretion of the veterinarian so long as maximum tolerable doses are not likely to be exceeded. The same repeating cycle can be repeated indefinitely until the veterinarian concludes that completion of any particular regimen has produced results sufficient to stop the treatments. Although negative results would understandably be very disappointing, it should be understood that a conclusion to stop the treatment at Decision Point **50** may involve results that sufficiently demonstrate that the FCGS will not likely respond to further multi-week administration regimens.

In addition to treatment of FCGS, alternative examples for some aspects of the disclosure are achieved by administering the same or analogous mTOR inhibitor regimens for prevention, treatment or management of conditions related to FCGS. For these purposes, conditions related to FCGS include not only FCGS but also any of the various forms of stomatitis and its precursors, concomitants and sequelae in feline subjects, as well as any of the various forms of stomatitis and its precursors, concomitants and sequelae in non-feline subjects. Still other examples are achieved by administering the same or analogous mTOR inhibitor regimens for prevention, treatment or management of related conditions such as any of the various forms of stomatitis and its precursors, concomitants and sequelae in non-feline animal subjects, which include human subjects in some alternatives. It will be understood, nonetheless, that variations in the dosing regimens described herein may be suitable and even beneficial depending on the species of animal of the subject, and also depending on the nature of the stomatitis-related condition for which the regimen is being applied.

Conditions that are considered related to FCGS include any and all of the various forms of stomatitis and its precursors, concomitants and sequelae.

Although preferred embodiments involve treatment of FCGS and related conditions, some aspects of the disclosure may be appreciated in the prevention, treatment and management of various other indications as well. are targeted in alternative embodiments, preferred embodiments of oral administration protocols according to the present invention are more stable, more bioavailable and efficacious, and find better biodistribution for treatment and prevention and reducing the progression of genetically-predisposed disorders and age-related disorders, with surprising benefits especially in the field of the prevention and treatment of gingivitis in felines and are thought to have analogous benefits in humans and other animals.

### PHARMACEUTICAL PREPARATIONS

Many of the methods of the present invention involve administration of MTOR inhibitors. Any inhibitor of mTOR is contemplated for inclusion in the present compositions and methods. In particular embodiments, the inhibitor of mTOR is rapamycin or an analog of rapamycin, preferably administered orally in the form of an enteric-coated rapamycin and/or e-nanoRapa preparation.

Rapamycin binds to a member of the FK binding protein (FKBP) family, FKBP 12. The rapamycin/FKBP 12 complex binds to the protein kinase mTOR to block the activity of signal transduction pathways. Because the mTOR signaling network includes multiple tumor suppressor genes, including PTEN, LKB1, TSC1, and TSC2, and multiple proto-oncogenes including P13K, Akt, and eEF4E, mTOR signaling plays a central role in cell survival and proliferation. Binding of the rapamycin/FKBP complex to mTOR causes arrest of the cell cycle in the G1 phase (Janus 2005).

mTOR inhibitors also include rapamycin analogs. Many rapamycin analogs are known in the art. Non-limiting examples of analogs of rapamycin include, but are not limited to, everolimus, tacrolimus, CC1-779, ABT-578, AP-23675, AP-23573, AP-23841, 7-epi-rapamycin, 7-thiomethyl-rapamycin, 7-epi-trimethoxyphenyl-rapamycin, 7-epi-thiomethyl-rapamycin, 7-demethoxy-rapamycin, 32-emethoxy-rapamycin, 2-desmethyl-rapamycin, and 42-O-(2-hydroxy)ethyl rapamycin.

Other analogs of rapamycin include: rapamycin oximes (U.S. Pat. No. 5,446,048); rapamycin aminoesters (U.S. Pat. No. 5,130,307); rapamycin dialdehydes (U.S. Pat. No. 6,680,330); rapamycin 29-enols (U.S. Pat. No. 6,677,357); O-alkylated rapamycin derivatives (U.S. Pat. No. 6,440,990); water soluble rapamycin esters (U.S. Pat. No. 5,955,457); alkylated rapamycin derivatives (U.S. Pat. No. 5,922,730); rapamycin amidino carbamates (U.S. Pat. No. 5,637,590); biotin esters of rapamycin (U.S. Pat. No. 5,504,091); carbamates of rapamycin (U.S. Pat. No. 5,567,709); rapamycin hydroxyesters (U.S. Pat. No. 5,362,718); rapamycin 42-sulfonates and 42-(N-carboalkoxy)sulfamates (U.S. Pat. No. 5,346,893); rapamycin oxepane isomers (U.S. Pat. No. 5,344,833); imidazolidyl rapamycin derivatives (U.S. Pat. No. 5,310,903); rapamycin alkoxyesters (U.S. Pat. No. 5,233,036); rapamycin pyrazoles (U.S. Pat. No. 5,164,399); acyl derivatives of rapamycin (U.S. Pat. No. 4,316,885); reduction products of rapamycin (U.S. Pat. Nos. 5,102,876 and 5,138,051); rapamycin amide esters (U.S. Pat. No. 5,118,677); rapamycin fluorinated esters (U.S. Pat. No. 5,100,883); rapamycin acetals (U.S. Pat. No. 5,151,413); oxorapamycins (U.S. Pat. No. 6,399,625); and rapamycin silyl ethers (U.S. Pat. No. 5,120,842).

Other analogs of rapamycin include those described in U.S. Pat. Nos. 6,015,809; 6,004,973; 5,985,890; 5,955,457; 5,922,730; 5,912,253; 5,780,462; 5,665,772; 5,637,590; 5,567,709; 5,563,145; 5,559,122; 5,559,120; 5,559,119; 5,559,112; 5,550,133; 5,541,192; 5,541,191; 5,532,355; 5,530,121; 5,530,007; 5,525,610; 5,521,194; 5,519,031; 5,516,780; 5,508,399; 5,508,290; 5,508,286; 5,508,285; 5,504,204; 5,491,231; 5,489,680; 5,489,595; 5,488,054; 5,486,524; 5,486,523; 5,486,522; 5,484,791; 5,484,790; 5,480,989; 5,480,988; 5,463,048; 5,446,048; 5,434,260; 5,411,967; 5,391,730; 5,389,639; 5,385,910; 5,385,909; 5,385,908; 5,378,836; 5,378,696; 5,373,014; 5,362,718; 5,358,944; 5,346,893; 5,344,833; 5,302,584; 5,262,424; 5,262,423; 5,260,300; 5,260,299; 5,233,036; 5,221,740; 5,221,670; 5,202,332; 5,194,447; 5,177,203; 5,169,851; 5,164,399; 5,162,333; 5,151,413; 5,138,051; 5,130,307; 5,120,842; 5,120,727; 5,120,726; 5,120,725; 5,118,678; 5,118,677; 5,100,883; 5,023,264; 5,023,263; 15,023,262. Additional rapamycin analogs and derivatives can be found in the following U.S. Patent Application Pub. Nos.: 20080249123; 20080188511; 20080182867; 20080091008; 20080085880; 20080069797; 20070280992; 20070225313; 20070203172; 20070203171; 20070203170; 20070203169; 20070203168; 20070142423; 20060264453; and 20040010002.

Rapamycin or a rapamycin analog can be obtained from any source known to those of ordinary skill in the art. The source may be a commercial source or a natural source. Rapamycin or a rapamycin analog may be chemically synthesized using any technique known to those of ordinary skill in the art. Non-limiting examples of information concerning rapamycin synthesis can be found in Schwecke *et al.,* 1995; Gregory *et al.*, 2004; Gregory *et al.*, 2006; Graziani, 2009.

Preferred embodiments of the present invention provide an improved form of encapsulated rapamycin - an encapsulated rapamycin nanoparticle that is more durable, stable and bioavailable, which enhances efficacy and predictability and ensures better biodistribution while also allowing improved patient compliance relative to raw rapamycin, as well as being produced at a reasonable cost. The improved form of encapsulated rapamycin preferably provides the rapamycin nanoparticles within a polymer matrix, forming an encapsulated rapamycin nanoparticle in a single drug delivery structure for oral administration of rapamycin. The polymer matrix, more particularly, is a controlled-release matrix, as described elsewhere in these descriptions. This encapsulated rapamycin nanoparticle may also be referred to as an enteric-coated rapamycin nanoparticle. In addition, many of the preferred embodiments also include a stabilizing compound (for our purposes, a "stabilizer") within the controlled-release matrix either to improve compatibility of the rapamycin with the controlled-release matrix, to stabilize the crystalline morphology of the rapamycin, or to help further prevent degradation of the rapamycin, particularly when the encapsulated rapamycin nanoparticle is exposed to air, atmospheric moisture, or room temperature or warmer conditions. Particularly preferred embodiments incorporate the stabilizers within each rapamycin nanoparticle, although certain aspects of the invention may be embodied with stabilizers on the surface of the encapsulated rapamycin nanoparticles or otherwise dispersed in the controlled-release matrix. To different levels depending on the particular approach used for producing the nanoparticles, with or without other additives, the result is more efficacious for treatment and prevention of in humans and other animals.

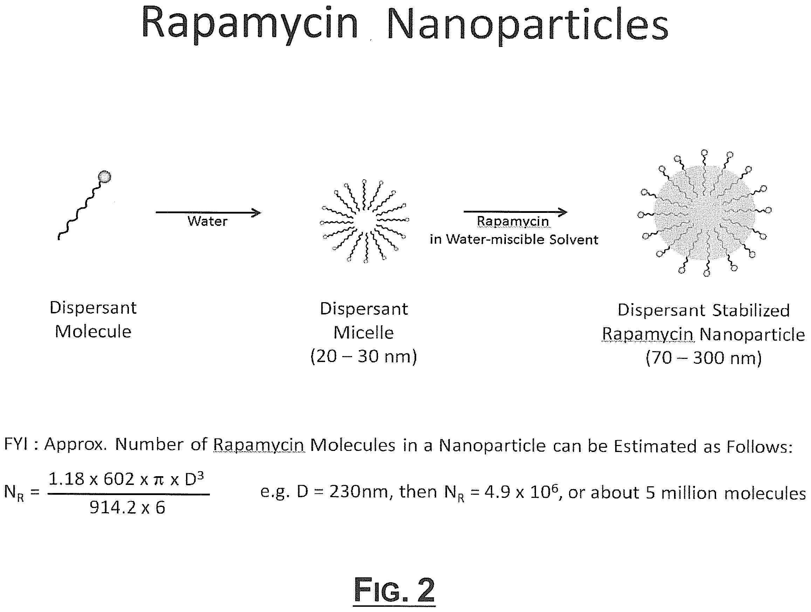

Rapid anti-solvent precipitation, or controlled precipitation, is a preferred method of preparing the rapamycin nanoparticles as it provides for minimal manipulation of the rapamycin and exquisite control over nanoparticle size and distribution, and the crystallinity of the rapamycin. Several controlled precipitation methods are known in the art, including rapid solvent exchange and rapid expansion of supercritical solutions, both of which can be implemented in batch or continuous modes, are scalable, and suitable for handling pharmaceutical compounds. Preferred embodiments use an anionic approach, producing micelles **130** (illustrated in **Fig. 2****)** or other molecular aggregations of amphipathic compounds (e.g. sodium cholate or similar surfactants with amphipathic tendencies) in concentrations greater than their critical micelle concentrations.

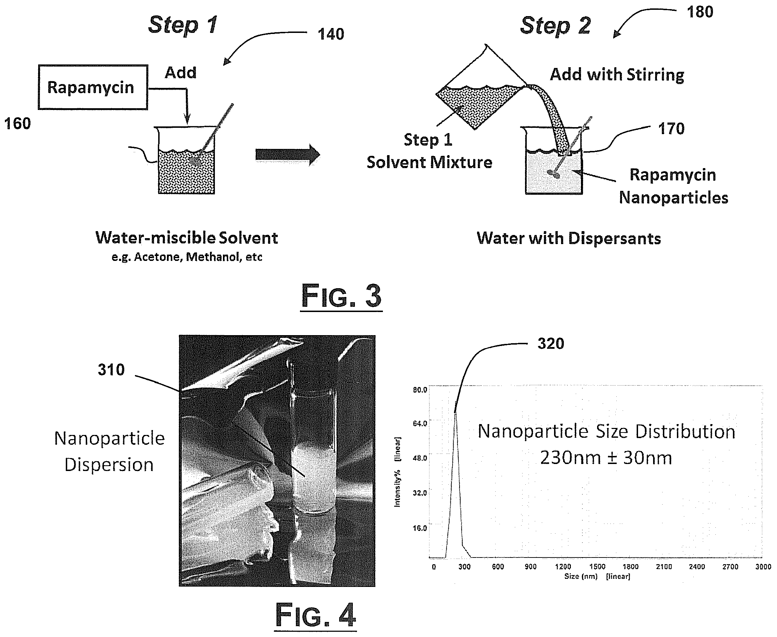

As part of a preferred process for producing microencapsulated rapamycin nanoparticles, **Figs. 2** and **3** illustrate basic preferred steps for producing a dispersion of preferred rapamycin nanoparticles through controlled precipitation. Rapamycin itself (sometimes referred to "raw" or "neat" rapamycin) is available in powder forms from multiple sources readily identifiable to those in the field. Although rapamycin is not readily soluble in water, solubility can be achieved in some aqueous miscible solvents.

Step 1 in **Fig. 3** illustrates a first basic step in the preferred process of producing preferred rapamycin nanoparticles, whereby raw rapamycin is mixed and dissolved into an aqueous miscible solvent **160** (the mixture represented by **140** in **Fig.1****).** As illustrated by Step 2 in **Fig. 3****,** the resultant solvent mixture is injected into rapidly stirred water containing an appropriate aqueous soluble dispersant **110,** preferably sodium cholate, which is a polar amphipathic molecule that tends to form micelles from solution.

After mixing the solvent mixture with the micelle-producing aqueous dispersant **110** in Step 2, the effects of solubility cause the rapamycin to partition to the hydrophobic micelle cores **130.** Appropriate solvents **160** and dispersants **110** are discussed in greater detail below. Although the core of the micelles **130** is relatively hydrophobic, which tends to attract the rapamycin from the solvent mixture, the results create a nanoparticle **150** having an outer surface decorated with hydrophilic ends of sodium cholate, which tend to keep the resulting nanoparticles in suspension within the final mixture.

A sample of a rapamycin nanoparticle dispersion **310** resulting from Step 2 is shown in the photograph in **Fig. 4****.** Alongside the photograph of **Fig. 4****,** a representative graph **320** is also provided. Graph **320** graphically shows an example of the resultant rapamycin nanoparticle size distribution in sample **310,** as indicated by the intensity of light (the vertical, ordinate axis in the graph) that is scattered by corresponding particle sizes (particle size being plotted as the horizontal abscissa axis in the graph) within the sample dispersion **310.** The use of sodium cholate as taught results in a hydrophilic surface, stabilizing the nanoparticles in the aqueous media and thereby preventing aggregation and particle growth. Alternative embodiments are also contemplated which use other amphoteric compounds as micelle-inducing compounds. The resulting nanoparticle products, as represented by sample 310, have properties that ensure enhanced and prolonged rapamycin stability - i.e., improved resistance to moisture degradation and/or oxidation for the final product - as well as good intestinal bioabsorption characteristics for the rapamycin protected in this manner.

Rapamycin nanoparticles prepared by controlled precipitation methods can be stabilized against irreversible aggregation, Ostwald ripening, and/or reduced dispersibility, by control of colloid chemistry, particle surface chemistry and particle morphology. For example, nanoparticles prepared by antisolvent solidification can be stabilized by ionic and non-ionic surfactants that adsorb to nanoparticle surfaces and promote particle colloid stability through either charge repulsion or steric hindrance, respectively. Moreover, stabilizers can affect nanoparticle crystallinity, which may be preferred to promote different biodistribution and bioavailability in certain indications.

Rapamycin nanoparticles can consist of molecular rapamycin bound by suitable methods to other nanoparticles. Suitable methods of attaching rapamycin to a nanoparticle carrier or substrate may include physical adsorption through hydrogen van der Waals forces or chemisorption through covalent or ionic bonding. Nanoparticle substrates may be either natural or synthetic, and modified to promote specific interactions with rapamycin. Natural nanoparticles include albumin and other proteins, and DNA. Synthetic nanoparticles include organic and inorganic particulates, micelles, liposomes, dendrimers, hyperbranched polymers, and other compounds.

The rapamycin nanoparticles can be processed by any suitable method, such as by milling, high pressure atomization, or rapid anti-solvent precipitation. Milling is suitable provided care is taken to minimize both rapamycin degradation and particle agglomeration. Rapamycin degradation can be reduced with the aid of cooling or cryogenic processes. Agglomeration due to the increased surface area and concomitant adhesive forces can be reduced by the use of dispersants **110** during the milling process.

The individual rapamycin nanoparticles are preferably sized in the range between about 1 nanometer and about 1 micron. Smaller sized rapamycin nanoparticles are preferred, preferably at less than 1 micron diameter, for various reasons, including better control of final particle size, improved stability within the particles, and the ability to tune bioavailability by controlling the crystallinity and composition of the rapamycin nanoparticles.

Manufacturing approaches for the encapsulated rapamycin nanoparticle drug delivery structure embodiments of the present invention include creating a solution of the controlled-release matrix, with the rapamycin nanoparticles dispersed therein, in appropriate proportion and producing a heterogeneous mixture. The solvent for such mixtures can be a suitable volatile solvent for the controlled-release matrix, although it is preferred the solvent be either a poor solvent or non-solvent for the rapamycin nanoparticles so that when the rapamycin nanoparticles are dispersed into the controlled-release matrix solution they remain as discrete nanoparticles. The resulting dispersion of rapamycin nanoparticles in the controlled-release matrix solution can then be reduced to a dry particulate powder by a suitable process, thereby resulting in microparticles of a heterogeneous nature comprised of rapamycin nanoparticles randomly distributed in the controlled-release matrix. The particulate powder may also be tailored by a suitable process to achieve a preferred particle size for subsequent preparation, which may be from about 20 to about 70 microns in diameter.

The rapamycin nanoparticles are microencapsulated with the controlled-release matrix using a suitable particle-forming process to form the encapsulated rapamycin nanoparticle. An example of a particle-forming process is spinning disk atomization and drying. For a detailed discussion of the apparatus and method concerning the aforementioned spin disk coating process see US Patent Applications 2011/221337 and 2011/220430. respectively. Alternatively, for example, the encapsulated rapamycin nanoparticles can be prepared by spray drying.

In some embodiments, not all of the rapamycin nanoparticles will be encapsulated within the controlled-release matrix. Instead the rapamycin nanoparticles may be enmeshed with the controlled-release matrix, with some of the rapamycin nanoparticles wholly contained within the controlled-release matrix while other rapamycin nanoparticles are apparent on the surface of the drug delivery structure, constructed in appearance similar to a chocolate chip cookie.

Depending on the size of the rapamycin nanoparticles, the encapsulated rapamycin nanoparticles are preferably of diameter between 10 and 50 microns, although diameters as large as 75 microns may be suitable for alternatives with corresponding compromises due to the larger size.

The controlled-release matrix of the encapsulated rapamycin nanoparticles can be selected to provide preferred release characteristics of the encapsulated rapamycin nanoparticles. For example, the matrix may be pH sensitive to provide either gastric release, or preferably, enteric release of the rapamycin. Enteric release of the rapamycin is preferred to achieve improved absorption and bioavailability of the rapamycin. Many materials suitable for enteric release are known in the art, including fatty acids, waxes, natural and synthetic polymers, shellac, and other materials. Polymers are a preferred enteric coating and may include copolymers of methacrylic acid and methyl methacrylate, copolymers of methyl acrylate and methacrylic acid, sodium alginate, polyvinyl acetate phthalate, and various succinate or phthalate derivatives of cellulose and hydroxypropyl methylcellulose. Synthetic polymers, such as copolymers of methacrylic acid and either methyl acrylate or methyl methacrylate, are preferred enteric release polymers due to the ability to tune the dissolution pH range of these synthetic polymers by adjusting their comonomer compositions. Examples of such pH sensitive polymers are EUDRAGIT^{®} polymers (Evonik Industries, Essen, Germany). Specifically, EUDRAGIT^{®} S 100, a methyl methacrylate and methacrylic acid copolymer with comonomer ratio of 2:1, respectively, has a dissolution pH of about 7.0, thereby making it suitable for enteric release of rapamycin.

The encapsulated rapamycin nanoparticles may be delivered in various physical entities including a pill, tablet, or capsule. The encapsulated rapamycin nanoparticles may be pressed or formed into a pellet-like shape and further encapsulated with a coating, for instance, an enteric coating. In another embodiment, the encapsulated rapamycin nanoparticles may be loaded into a capsule, also further enterically coated.

Various performance enhancing additives can be added to the encapsulated rapamycin nanoparticles. For example, additives that function as free radical scavengers or stabilizers can be added to improve oxidative and storage stability of the encapsulated rapamycin nanoparticles. Free radical scavengers are preferably chosen from the group that consists of glycerol, propylene glycol, and other lower alcohols. Additives alternatively incorporate antioxidants, such as α-tocopherol (vitamin E), citric acid, EDTA, α-lipoic acid, or the like.

Methacrylic acid copolymers with methyl acrylate or methyl methacrylate are moderate oxygen barriers. Furthermore, these polymers will exhibit an equilibrium moisture content. Oxygen transport due to residual solvent, moisture or other causes, can lead to degradation of the encapsulated rapamycin nanoparticles. Oxygen barrier materials can be added to the encapsulated rapamycin nanoparticles formulation to improve oxygen barrier properties. Preferred oxygen barrier polymers compatible with the preferred polymers are polyvinyl alcohol (PVA) and gelatin.

### PREFERRED MICROPARTICLE AND NANOPARTICLE EMBODIMENTS

Preferred embodiments with rapamycin nanoparticle inclusions comprise discrete nanoparticles of rapamycin heterogeneously dispersed in a controlled-release matrix. As illustrated in accompanying drawings, the rapamycin nanoparticles are prepared by a suitable method and may contain additives to promote nanoparticle stability, modify rapamycin crystallinity, or promote compatibility of the rapamycin nanoparticles with the controlled-release matrix. The controlled-release matrix is formulated to promote release of rapamycin to specific parts of the body, such as the intestine, to enhance oxidative and storage stability of the encapsulated rapamycin nanoparticles, and to maintain the discrete, heterogeneously distributed nature of the rapamycin nanoparticles.

Rapamycin nanoparticles are preferably prepared by anti-solvent precipitation or solidification, also sometimes referred to as controlled precipitation or solidification. Antisolvent solidification is a preferred approach as it provides exquisite control of particle size and distribution, particle morphology, and rapamycin crystallinity. For example, it is possible to prepare nanoparticles with narrow particle size distribution that are amorphous, crystalline, or combinations thereof. Such properties may exhibit additional benefits, by further controlling the biodistribution and bioavailability of rapamycin in specific indications.

Rapamycin is dissolved in a suitable water-miscible solvent **160** and then rapidly injected into rapidly stirred water containing an appropriate aqueous soluble dispersant **110.** Water-miscible solvents **160** for rapamycin include methanol, ethanol, isopropyl alcohol, acetone, dimethylsulfoxide, dimethylacetamide, n-methylpyrolidone, tetrahydrofuran, and other solvents. Low boiling point, high vapor pressure water-miscible solvents **160** are preferred to facilitate their removal during subsequent microparticle formation. Some preferred water-miscible solvents **160** are methanol, acetone, and isopropyl alcohol. A preferred water-miscible solvent **160** is methanol. Some aqueous soluble dispersants **110** include ionic surfactants such as sodium dodecyl sulfate and sodium cholate, non-ionic surfactants such as Pluronics, Poloxomers, Tweens, and polymers, such as polyvinyl alcohol and polyvinylpyrolidone. Some preferred aqueous-soluble dispersants **110** are sodium cholate, Pluronic F-68, and Pluronic F-127. A preferred aqueous-soluble dispersant **110** is sodium cholate, which provides surprisingly beneficial properties in the present application.

Not only is sodium cholate a surfactant and a dispersant, it serves to produce multimolecular structures which tend to cause aggregation of rapamycin within those structures, particularly when the pH and other condition of the aqueous solution are controlled to allow aggregation of the rapamycin from that aqueous solution. The resulting process allows for rapamycin nanoparticle production that not only tends to produce nanoparticles in highly predictable size ranges, but also provides a resulting nanoparticle with surprisingly desirable levels of colloidal stability. Moreover, while sodium cholate tends to be a polar molecule as well as an amphoteric surfactant, it induces an ionic charge in each hydrophilic nanoparticle when it is enmeshed in the EUDRAGIT^{®} matrix. It is believed that when the nanoparticle is released from the EUDRAGIT^{®} matrix within the animal subject's enteric passages where conditions are basic, the same properties cause the nanoparticle to be more readily received and absorbed through the intestinal walls.

Rapamycin is dissolved in the water-miscible solvent **160** at a concentration of about 0.01% w/v to about 10.0% w/v preferably about 0.1% w/v to about 1.0% w/v. The aqueous-soluble dispersant **110** is dissolved in water at a concentration above its critical micelle concentration, or CMC, typically at about 1 to about 10 times the CMC. The rapamycin solution is injected into the aqueous-soluble dispersant solution with agitation at a volumetric ratio of about 1:10 to about 1:1, preferably about 1:5 to about 1:1.

The controlled-release matrix is prepared from a water-soluble polymer, preferably a copolymer of methacrylic acid with either methyl acrylate or methyl methacrylate, such as those marketed under the trade name of EUDRAGIT^{®} and having pH-dependent dissolution properties. More preferably the controlled-release matrix is comprised of EUDRAGIT^{®} S 100, although other water-soluble enteric controlled release would be suitable. Water-soluble controlled-release matrices are selected so as either not to compromise the integrity of rapamycin nanoparticles or to provide a medium in which rapamycin nanoparticles may be prepared by the controlled precipitation methodology described previously.

In preparing the water-soluble polymer it is preferable to maintain conditions that do not compromise the integrity of the rapamycin nanoparticles. Firstly, since the rapamycin nanoparticles are susceptible to solubilization by certain co-solvents, it is important to maintain a suitable quantity of certain co-solvents to achieve controlled-release matrix solubility while not deleteriously affecting the morphology of the rapamycin nanoparticles. Secondly, rapamycin nanoparticles will be susceptible to chemical degradation by high pH; therefore, it is important to modulate the controlled-release matrix solution pH so that rapamycin is not chemically altered. It is preferable the controlled-release matrix solution pH be maintained below about pH 8. Lastly, it is preferable to achieve near to complete solubilization of the controlled-release matrix in solution so that microencapsulation of the rapamycin nanoparticles by the controlled-release matrix in subsequent processing steps may proceed with high efficiency. When using the EUDRAGIT^{®} S 100 as the controlled-release matrix, it is preferable to achieve a controlled-release matrix solution by using a combination of co-solvents and solution pH modulation. It is preferable the co-solvents be about 40% or less by volume. Similarly, it is preferable that the pH of the controlled-release matrix solution be about 8 or less, such that the EUDRAGIT^{®} S 100 is not completely neutralized and is preferably only about 80% or less neutralized. These preferred conditions achieve nearly complete to complete solubilization of the EUDRAGIT^{®} S 100 in a medium that is mostly aqueous and that maintains the integrity of the rapamycin nanoparticles, therefore leading to their microencapsulation by the controlled-release matrix in subsequent processing steps.

The rapamycin nanoparticles prepared by the preferred controlled precipitation method are added to the aqueous solution of the controlled-release matrix, resulting in a nanoparticle dispersion in the solubilized controlled-release matrix. Alternatively, the rapamycin solubilized in a suitable or preferred co-solvent can be dispersed into the aqueous solution of controlled-release matrix leading to controlled precipitation of rapamycin particles, thereby leading to a rapamycin nanoparticle dispersion in fewer processing steps, but of appropriate composition to permit subsequent microencapsulation processing.

As an alternative embodiment, the encapsulated rapamycin nanoparticles are created using pre-existing nanoparticle substrates, such as albumin, to create, in the case of albumin, "albumin-rapamycin nanoparticles." Within this general class of alternatives, preferred approaches for creating the album in-rapamycin nanoparticles involve encapsulating rapamycin within albumin nanoparticles or preferentially associating rapamycin with albumin nanoparticles through physical or chemical adsorption. The albumin nanoparticles themselves are preferably formed from human serum albumin, a plasma protein derived from human serum.

More particularly, this embodiment preferably involves use of a therapeutic peptide or protein that is covalently or physically bound to albumin, to enhance its stability and half-life. With the albumin stabilized, the rapamycin is mixed with the stabilized albumin in an aqueous solvent and passed under high pressure to form rapamycin-albumin nanoparticles in the size range of 100-200 nm (comparable to the size of small liposomes).

Preferred embodiments also address degradation risks and other limits imposed by the related art by preparing encapsulated rapamycin nanoparticles as a heterogeneous mixture of rapamycin nanoparticles in a polymer matrix. Distributed nanoparticles are morphologically different than homogeneous rapamycin and are less susceptible to degradation because of the bulk nature of the nanoparticles compared to the smaller size of molecular rapamycin.

Another alternative embodiment involves biodegradable polymers loaded with rapamycin. Biodegradable polymers loaded with drugs can be microparticles. "Microparticle" refers to particles between about 0.1 and 300 µm in size. Drug-loaded biodegradable polymers release the drug in a time-dependent manner.

As used herein, "biodegradable" refers to any natural means by which a polymer can be disposed of in a patient's body. This includes such phenomena as, without limitation, biological decomposition, bioerosion, absorption, resorption, etc. Biodegradation of a polymer in vivo results from the action of one or more endogenous biological agents and/or conditions such as, without limitation, enzymes, microbes, cellular components, physiological pH, temperature and the like.

In some aspects, the biodegradable polymers can be poly-ε-caprolactone (PCL) microparticles. PCL is a biodegradable, biocompatible, and semicrystalline polymer. PCL is useful for drug delivery because it is highly permeable to many drugs and is non-toxic. Sinha *et al.* 2004. Rapamycin can also be loaded onto microparticles of other biodegradable polymers, including but not limited to aliphatic polyester, polylactide, polyglycolide, poly(lactide-co-glycolide), mixtures thereof, and their copolymers. Such biodegradable polymers are known in the art.

Rapamycin may be loaded onto microspheres of PCL alone or of PCL copolymers or blends to obtain the desired drug release characteristics. Copolymers of PCL can be formed using many different monomers, including, but not limited to, ethyleneoxide, polyvinylchloride, chloroprene, polyethylene glycol, polystyrene, diisocyanates (urethanes), tetrahydrofuran (THF), diglycolide, dilactide, δ-valeractone, substituted caprolactones, 4-vinyl anisole, styrene, methyl methacrylate, and vinyl acetate.

Drug-loaded PCL microspheres can be prepared by several different methods known by persons of skill in the art, including, but not limited to, an o/w emulsion solvent extraction/evaporation method, a w/o/w emulsion solvent evaporation technique, a spray drying technique, a solution-enhanced dispersion method, and a hot melt technique. These methods are described in more detail in Sinha *et al.,* 2004, which is hereby incorporated by reference. Briefly, as a non-limiting example, the o/w emulsion solvent extraction evaporation method can be performed by dissolving the required amount of polymer and drug in an organic phase, emulsifying under stirring with polyvinyl alcohol to form an o/w emulsion, stirring for 3 hours at about 500 rpm to evaporate the organic phase, and filtering and drying the formed microspheres.

Drug-loaded microspheres of aliphatic polyesters, polylactide, polyglycolide, and poly(lactide-co-glycolide) can be prepared by several different methods known by persons of skill in the art. Non-limiting examples can be found in the following references: Kemala *et al.,* 2012; Ghassemi *et al.,* 2009; Corrigan & Heelan, 2001; Cleland et al., WIPO Pub. No. WO 1995/11009; and Atkins et al., WIPO Pub. No. WO 1995/009613.

In some aspects of this alternative embodiment, the microparticles loaded with rapamycin are encased, encapsulated, or coated to provide for release in the intestinal tract, including the colon.

In some aspects, the microparticles are coated with an enteric coating, which is a coating that prevents release and absorption of active ingredients until they reach the intestine. Some enteric coatings facilitate delivery of agents to the colon. In some embodiments, the enteric coating is a EUDRAGIT^{®} coating. EUDRAGIT^{®} coatings include EUDRAGIT^{®} L 100-55, Poly(methacrylic acid-co-ethyl acrylate) 1:1; EUDRAGIT^{®} L 30 D-55, Poly(methacrylic acid-co-ethyl acrylate) 1:1; EUDRAGIT^{®} L-100, Poly(methacrylic acid-co-methyl methacrylate) 1:1; EUDRAGIT^{®} S 100, Poly(methacrylic acid-co-methyl methacrylate) 1:2; EUDRAGIT^{®} FS 30 D, Poly(methyl acrylate-co-methyl methacrylate-co-methacrylic acid) 7:3:1; EUDRAGIT^{®} RL, Poly(ethyl acrylate-co-methyl methacrylate-co-trimethylammonioethyl methacrylate chloride) 1:2:0.2; EUDRAGIT^{®} RS, Poly(ethyl acrylate-co-methyl methacrylate-co-trimethylammonioethyl methacrylate chloride) 1:2:0.1; and EUDRAGIT^{®} E, Poly(butyl methacrylate-co-(2-dimethylaminoethyl) methacrylate-co-methyl methacrylate) 1:2:1. Other coatings include EUDRAGIT^{®} RS, EUDRAGIT^{®} RL, ethylcellulose, and polyvinyl acetate. Benefits include pH-dependent drug release, protection of active agents sensitive to gastric fluid, protection of gastric mucosa from active agents, increase in drug effectiveness, good storage stability, and GI and colon targeting, which minimizes risks associated with negative systemic effects and maintains effective dosing.

In some aspects, colon targeting of rapamycin can be achieved by creating PCL microparticles loaded with rapamycin or rapamycin analog and subsequently coating the microparticles with EUDRAGIT^{®} S 100. Methods of making such coated microparticles can be found in Ghorab *et al.,* 2011, which is hereby incorporated by reference. Briefly, drug-loaded PCL microparticles are suspended in a solution containing an appropriate amount of EUDRAGIT^{®} S 100 dissolved in ethyl alcohol. The suspension is poured into distilled water. The resulting mixture is homogenized for five minutes and then mechanically stirred until the organic solvent is completely evaporated. Microparticles are collected, washed with cyclohexane twice, and dried overnight in a dessicator.

Some other examples of enteric coating components include cellulose acetate phthalate, methyl acrylate-methacrylic acid copolymers, cellulose acetate succinate, hydroxypropyl methylcellulose phthalate, hydroxypropyl methylcellulose acetate succinate, polyvinyl acetate phthalate, methyl methacrylate-methacrylic acid copolymers, sodium alginate, and stearic acid. The coating may include suitable hydrophilic gelling polymers including, but not limited to, cellulosic polymers, such as methylcellulose, carboxymethylcellulose, hydroxypropylcellulose, hydroxypropylmethylcellulose, hydroxyethylcellulose, and the like; vinyl polymers, such as polyvinylpyrrolidone, polyvinyl alcohol, and the like; acrylic polymers and copolymers, such as acrylic acid polymer, methacrylic acid copolymers, ethyl acrylate-methyl methacrylate copolymers, natural and synthetic gums, such as guar gum, arabic gum, xanthan gum, gelatin, collagen, proteins, polysaccharides, such as pectin, pectic acid, alginic acid, sodium alginate, polyaminoacids, polyalcohols, polyglycols, and the like; and mixtures thereof. Any other coating agent known to those of ordinary skill in the art is contemplated for inclusion in the coatings of the microcapsules set forth herein.

The coating may optionally comprise a plasticizer, such as dibutyl sebacate, polyethylene glycol and polypropylene glycol, dibutyl phthalate, diethyl phthalate, triethyl citrate, tributyl citrate, acetylated monoglyceride, acetyl tributyl citrate, triacetin, dimethyl phthalate, benzyl benzoate, butyl and/or glycol esters of fatty acids, refined mineral oils, oleic acid, castor oil, corn oil, camphor, glycerol and sorbitol or a combination thereof. The coating may optionally include a gum. Non-limiting examples of gums include homopolysaccharides such as locust bean gum, galactans, mannans, vegetable gums such as alginates, gum karaya, pectin, agar, tragacanth, acacia, carrageenan, chitosan, alginic acid, other polysaccharide gums (e.g., hydrocolloids), acacia catechu, salai guggal, indian bodellum, sopaiba gum asafetida, cambi gum, *Enterolobium cyclocarpum,* mastic gum, benzoin gum, sandarac, gambier gum, butea frondosa (Flame of Forest Gum); myrrh, konjak mannan, guar gum, welan gum, gellan gum, tara gum, locust bean gum, carrageenan gum, glucomannan, galactan gum, sodium alginate, xanthan gum deacetylated xanthan gum, pectin, sodium polypectate, gluten, karaya gum, tamarind gum, ghatti gum, Acaroid/Yacca/Red gum, dammar gum, juniper gum, ester gum, ipil-ipil seed gum, gum talha (acacia seyal), and cultured plant cell gums including those of the plants of the genera: *Acacia, Actinidia, Aptenia, Carbobrotus, Chickorium, Cucumis, Glycine, Hibiscus, Hordeum, Letuca, Lycopersicon, Malus, Medicago, Mesembryanthemum, Oryza, Panicum, Phalaris, Phleum, Poliathus, Polycarbophil, Sida, Solanum, Trifolium, Trigonella, Afzelia africana* seed gum, *Treculia africana* gum, detarium gum, cassia gum, carob gum, *Prosopis africana* gum, *Colocassia esulenta* gum, *Hakea gibbosa* gum, khaya gum, scleroglucan, zea, mixtures of any of the foregoing, and the like.

A variety of other encasing materials and systems for delivering rapamycin-loaded biodegradable microspheres to the colon can be used alone or in combination with a pH-dependent coating like EUDRAGIT^{®} S 100. Non-limiting examples follow.

Hydrophilic gelling polymers or copolymers can be included in a material encasing one or more microspheres to provide a time-dependent release of drug-loaded microspheres. Non-limiting examples of hydrophilic gelling copolymers include methylcellulose, carboxymethylcellulose, hydroxypropylcellulose, hydroxypropyl methylcellulose, carbomers, polyvinyl alcohols, polyoxyethylene glycols, polyvinylpyrrolidones, poloxamers, or natural or synthetic rubbers. An intermediate layer of these polymers can be included to delay release of active ingredient for a desired amount of time, as described in Poli et al., (EP0572942). Another example of a time-dependent encasing material is a wax matrix including, for example, behenic acid, as described in Otuska & Matsuda, 1994.

Polysaccharides that are resistant to digestive enzymes but are enzymatically broken down by bacteria in the colon can be included in an encasing material. Non-limiting examples include chitosan and pectin as described in Coulter (EP2380564), and azopolymers, disulfide polymers, amylose, calcium pectinate, and chondroitin sulfate as described in Watts (EP0810857).

A starch capsule coated with an enteric coating such as EUDRAGIT^{®} S 100 or EUDRAGIT^{®} L 100 may be used, as described in Watts (EP0180857). A variety of starches, including modified starches and starch derivatives may be used. Non-limiting examples include hydroxyethyl starch, hydroxypropyl starch, carboxymethyl starch, cationic starch, acetylated starch, phosphorylated starch, succinate derivatives, or grafted starches.

A layer of insoluble or relatively insoluble rupturable polymer can be used as part of a strategy to provide for abrupt release of drug-loaded microspheres in the colon. The rupturable polymer can comprise one or more of a variety of suitable polymers known by those of skill in the art, including, but not limited to, cellulose acetate, cellulose acetate propionate, or ethyl cellulose. A variety of strategies for causing rupture of the polymer in the colon can be employed. As a non-limiting example, the rupturable polymer can be designed to rupture upon encountering increased pressure due to intestinal peristalsis, as described in Muraoka *et al.*, 1998. As another example, the rupturable polymer can be semi-permeable, and an effervescent solid can be included in a core containing the drug-loaded microparticles, as described in Krogel & Bodmeier, 1999. As another example, a layer of swellable material, including, but not limited to, croscarmellose sodium or hydroxypropyl methylcellulose, can be disposed within the rupturable polymer layer, as described in Bussemer *et al.,* 2001. Controlled entry of water past the rupturable polymer layer can be provided by embedded hydrophilic particulate material, as described in Lerner et al., (WIPO Pub. No. WO 1999/018938).

A two-piece encasing system, as described in McNeill et al., (WIPO Pub. No. WO 1990/009168) can be used to provide for release of drug-loaded microspheres in the colon. One of the pieces is a relatively water insoluble capsule with an open orifice, which is covered by a second piece that swells as it takes up water. The swelling causes displacement from the orifice and release of the capsule contents.

### EXAMPLES OF PREFERRED MTOR INHIBITING PREPARATIONS

Example 1 - Development of methods to produce rapamycin nanoparticles. Rapid solvent exchange was used to examine the formation of rapamycin nanoparticles. Three water-miscible solvents **160** and three water-soluble surfactants were selected to study their respective effects on the formation and morphology of rapamycin nanoparticles. The water-miscible solvents **160** were isopropyl alcohol (Solvent 1), acetone (Solvent 2), and methanol (Solvent 3). The water-soluble surfactants were Pluronic F-68 (Dispersant 1, a non-ionic PEO-PPO-PEO block copolymer), Pluronic F-127 (Dispersant 2, a non-ionic PEO-PPO-PEO block copolymer), and sodium cholate (Dispersant 3, an anionic surfactant). Rapamycin was dissolved in each of the water-miscible solvents **160** at a concentration of 0.25% w/v. The water-soluble surfactants were dissolved in deionized water at concentrations of 0.5% w/v, 0.5% w/v, and 1.0% w/v, respectively, for each of the dispersants. Each experimental combination (e.g. NP-1 to NP-9 in following table) consisted of 5mL of rapamycin solution and 25mL of surfactant solution, resulting in a dilution factor of 1:5 solvent:water. 25mL of surfactant solution was transferred to a 50mL beaker and stirred with the aid of magnetic micro stir bar. Rapamycin solution was rapidly injected at 500µL increments with the aid of a micropipette with the pipette tip placed below the surface of the rapidly stirred surfactant solution. The visual appearance of the resulting nanoparticles and their colloidal stability after 24-hours were qualitatively assessed. The following table summarizes the qualities of the rapamycin nanoparticle dispersions. Qualitatively, rapamycin nanoparticle dispersions having a colorless to blue, opalescent appearance will have particle sizes on the order of less than about 300nm as evidenced by their interaction with the ultraviolet wavelengths of visible light. Whereas, dispersions having a more white appearance will have particle sizes larger than about 300nm due to their interaction with the broader spectrum of visible light. Rapamycin nanoparticle formulations NP-7 and NP-9 were selected as preferred methods of nanoparticle preparation.

| | Dispersant 1 | Dispersant 2 | Dispersant 3 |
|---|---|---|---|
| Solvent 1 | NP-1 White, settled, resdispersible | NP-2 Blue, opalescent, settled, redispersible | NP-3 Clear, aggregated, redispersible |
| Solvent 2 | NP-4 Blue, opalescent, some settling | NP-5 White, settled, redispersible | NP-6 Blue, opalescent, settled, redispersible |
| Solvent 3 | NP-7 Blue, opalescent, stable | NP-8 : Blue to white, settled, redispersible | NP-9 Blue, opalescent, stable |

Example 2 - Preparation of a high concentration rapamycin nanoparticle dispersion. The water-miscible solvent **160** and water-soluble dispersant **110** of NP-9 from Example 1 was used to prepare rapamycin nanoparticles. 656mg of rapamycin were dissolved in 6.56mL of Solvent 3 to yield a 1.0% w/v solution. This volume of rapamycin solution was injected into 26.25mL of 1.0% w/v Dispersant 1 in deionized water. The resulting rapamycin nanoparticle dispersion had a final rapamycin content of 2.4% w/w. The particle size of the dispersion was determined by dynamic light scattering to be 230nm ± 30nm with a single peak.

Example 3 - Preparation of a water-soluble enteric coating. 3.5g of EUDRAGIT^{®} S 100 were added to 70mL of deionized water with light stirring, resulting in a white dispersion. 1.4g of sodium hydroxide were added to the dispersion with continued stirring. The resulting dispersion gradually turned clear and colorless indicating an aqueous solution of S-100. The estimated concentration of sodium hydroxide was 0.5M.

Example 4 - Preparation of a feedstock containing rapamycin nanoparticles and a water-soluble enteric coating. Rapamycin nanoparticles were prepared as described in Example 2 and then slowly added to an aqueous solution of EUDRAGIT^{®} S 100 prepared as in Example 3. The ratio of rapamycin to EUDRAGIT^{®} S 100 was 1:9, or 10% wt. rapamycin payload. The resulting dispersion was allowed to stir for several minutes to observe stability. After one hour, the dispersion had transformed to a clear yellow, indicating destruction of the rapamycin nanoparticles and a change in the rapamycin. Addition of a small amount of acetic acid to reduce the solution pH to below neutral resulted in a clear, colorless solution.

Example 5 - Preparation of water-soluble enteric coating with a water-miscible co-solvent. 3.5g of EUDRAGIT^{®} S 100 were added to 30/70 v/v methanol/deionized water, resulting in a white dispersion. The dispersion was stirred continuously until a clear solution was formed.

Example 6 - Preparation of a feedstock containing rapamycin nanoparticles and a water-soluble enteric coating. Rapamycin nanoparticles were prepared as described in Example 2 and then slowly added to an aqueous solution of EUDRAGIT^{®} S 100 prepared as in Example 5. The ratio of rapamycin to S 100 was 1:9, or 10% wt. rapamycin payload. The white dispersion was allowed to stir for several minutes after which the dispersion was transformed into a clear solution indicating the rapamycin nanoparticles had been destroyed.

Example 7 - Preparation of a partially-neutralized, water-soluble enteric coating with a water-miscible co-solvent. 3.5g of EUDRAGIT^{®} S 100 were added to 10/90 v/v methanol/deionized water, resulting in a white dispersion. The dispersion was titrated to clarity with 2.000mL of 4.8M sodium hydroxide. The estimated neutralization of the S-100 was 78%.

Example 8 - Preparation of a feedstock containing rapamycin nanoparticles and a water-soluble enteric coating. Rapamycin nanoparticles were prepared as described in Example 2 then slowly added to an aqueous solution of EUDRAGIT^{®} S 100 as prepared in Example 7. The ratio of rapamycin to EUDRAGIT^{®} S 100 was 1:9, or 10% wt. rapamycin payload. The resulting white dispersion remained stable for several hours as indicated by no change in color or change in optical clarity. The final pH was 7.5. The particle size of the final dispersion was determined by dynamic light scattering to be 756nm ± 52nm with a single peak and indicating possible clustering of the rapamycin nanoparticles in the resulting feedstock.

Example 9 - Preparation of a feedstock containing rapamycin nanoparticles and a water-soluble enteric coating. The rapamycin solution used in Example 2 was injected, with stirring, into the aqueous solution of EUDRAGIT^{®} S 100 prepared in Example 7. The ratio of rapamycin to EUDRAGIT^{®} S 100 was 1:9, or 10% wt. rapamycin payload. A blue, opalescent colloid was formed and it remained stable for several hours as indicated by no change in color or change in optical clarity. The final pH was 7.5. The particle size of the final dispersion was determined by dynamic light scattering to be 305nm ± 60nm with a single peak.

Example 10 - Spray drying of feedstock containing rapamycin nanoparticles and a water-soluble enteric coating. The feedstocks prepared in Examples 8 and 9 were spray dried and analyzed for rapamycin content. Particles prepared from Example 8 had a rapamycin content of 9.5% wt. (87% rapamycin yield). Particles prepared from Example 9 had a rapamycin content of 7.9% wt. (80% rapamycin yield).

Example 11 - Storage stability of enteric-coated encapsulated rapamycin nanoparticles. Microparticles prepared by spray drying in Example 10 were stored under controlled conditions at room temperature and 50% relative humidity. Samples were analyzed weekly for rapamycin content. All samples maintained at least 95% of their original rapamycin content at all time points for at least three weeks.

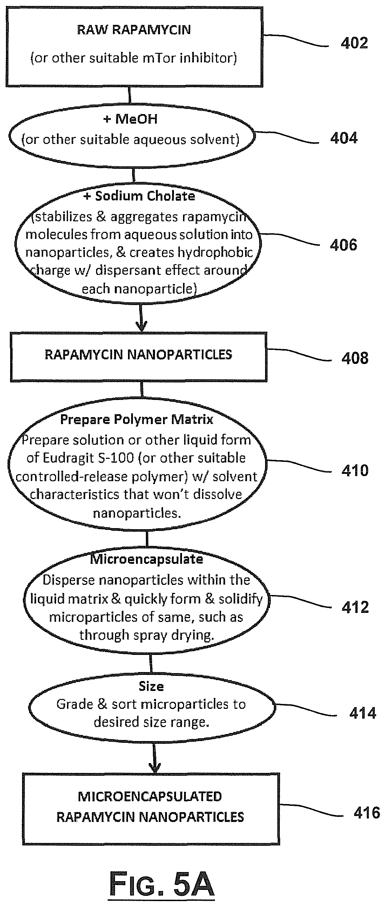

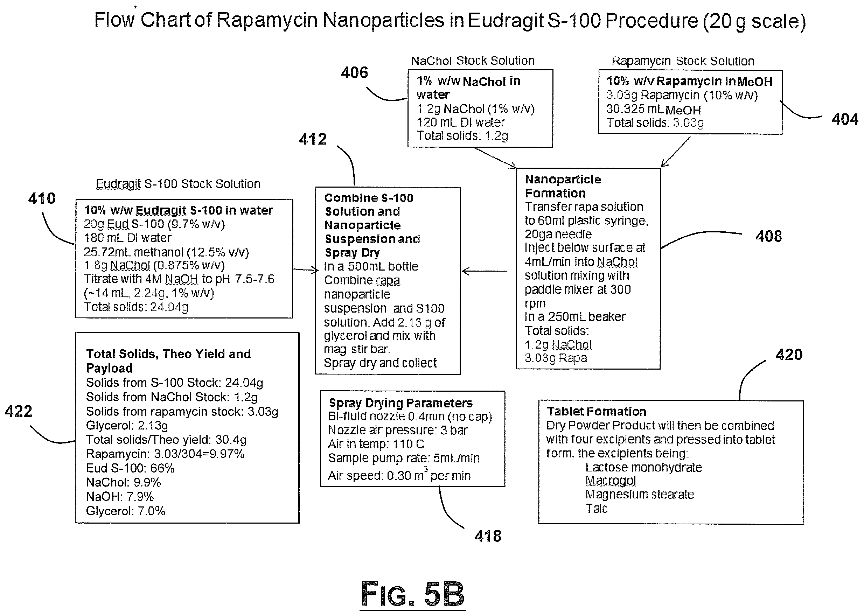

Example 12 - Preparation of nanoparticles in EUDRAGIT^{®} S 100 as illustrated in **Figs. 5A** and **5B****.** A rapamycin solution was prepared by combining rapamycin with methanol (at Steps **402** and **404)** in a 10% w/v ratio as 3.03g rapamycin and 30.25mL methanol. A 1% w/w sodium cholate solution was prepared by combining 1.2g sodium cholate with 120mL deionized water as shown in Step 424. Nanoparticle formation was achieved by transferring the rapamycin solution with a 60mL plastic syringe equipped with a 20ga needle and, injecting the rapamycin solution below the surface of the sodium cholate solution in a 250 mL beaker (Steps **406** and **408).** Mixing was accomplished with a paddle mixer operating at 300rpm yielding a rapamycin nanoparticle suspension. At Step **410,** a 10% w/w EUDRAGIT^{®} S 100 solution was prepared by combining 20g EUDRAGIT^{®} S 100 in a 9.7% w/v mixture with 180mL deionized water, 25.72mL methanol in a 12.5% v/v mixture, and 1.8g sodium cholate in a 0.875% w/v mixture. This 10% w/w EUDRAGIT^{®} S 100 solution was titrated with 4M sodium hydroxide to achieve a pH of between about 7.5 and about 7.6. Encapsulated rapamycin particles were then fabricated by combining the EUDRAGIT^{®} S 100 solution with the rapamycin nanoparticle suspension at Step **412.** The EUDRAGIT^{®} S 100 solution and the rapamycin nanoparticle suspension were combined in a 500mL bottle, adding 2.13g of glycerol and mixing with a magnetic stir bar. The combined EUDRAGIT^{®} S 100 solution and rapamycin nanoparticle suspension were then spray dried and collected. The spray drying parameters (shown at Step **418)** included a 0.4mm nozzle, nozzle air pressure of 3 bar, input air temperature of 110°C, a sample pump rate of 5mL/min and an air speed of 0.30 m/min. After the preferred nanoparticle microencapsulation process is complete, the nanoparticles may then be graded and sorted according to the desired size range at Step **414.** Alternatively, the resulting dispersion of rapamycin nanoparticles in the controlled-release matrix solution can be reduced to a dry particulate powder by a suitable process, thereby resulting in microparticles of a heterogeneous nature comprised of rapamycin nanoparticles randomly distributed in the controlled-release matrix. This dry particulate powder can then be combined with excipients and pressed into tablet form as indicated at Step **420.**

Preferred embodiments of the tablet form for oral administration of eRapaNP2g may include between 1 and 3 mg of pharmaceutically active rapamycin, with the tablets preferably constituting approximately 10% by weight of rapamycin as shown in Step **422.** Also as indicated in Step **422,** the preferred dispersant used is sodium cholate at approximately 10% by weight in the final product. EUDRAGIT^{®} S 100 functions as the enteric release co-polymer, preferably at approximately 65% by weight. Sodium hydroxide is preferably used to neutralize the EUDRAGIT^{®} S 100 as previously described, with no sodium hydroxide being present in the final product. Various components may be utilized as excipients in the final preparation as described above. As shown in Step **420,** preferred embodiments preferably use excipients including lactose monohydrate, macrogol or polyethylene glycol (PEG 8000), magnesium stearate, and talc. The amount of each excipient by weight is variable dependent on production of an effective tablet. However, lactose monohydrate and PEG 8000 preferably constitute the bulk of the excipients by weight and percentage in the entire tablet in preferred embodiments, with each constituting 35-50% of the preferred tablet composition. This percentage would equate to approximately 55-70 mg of each of these two components in preferred embodiments. The remaining excipients, magnesium stearate and talc, would constitute less of the final composition, each being approximately 1% by weight of the entire tablet in preferred embodiments.

### METHODS OF USING RAPAMYCIN COMPOSITIONS

"Treatment" and "treating" refer to administration or application of a therapeutic agent to a subject or performance of a procedure or modality on a subject for the purpose of obtaining a therapeutic benefit for a disease or health-related condition. For example, the rapamycin compositions of the present invention may be administered to a subject for the purpose of treating or preventing FCGS.

The terms "therapeutic benefit," "therapeutically effective," or "effective amount" refer to the promotion or enhancement of the well-being of a subject. This includes, but is not limited to, a reduction in the frequency or severity of the signs or symptoms of a specified malady or group of maladies.

"Prevention" and "preventing" are used according to their ordinary and plain meaning. In the context of a particular disease or health-related condition, those terms refer to administration or application of an agent, drug, or remedy to a subject or performance of a procedure or modality on a subject for the purpose of preventing or delaying the onset of a disease or health-related condition.

Rapamycin compositions, as disclosed herein, including preferably encapsulated rapamycin nanoparticles, may be used to prevent, treat, delay or reduce any disease or condition (or its precursors or sequelae) for which an inhibitor of mTOR is contemplated as effective for treatment, prevention, or delaying or reducing its progression. For example, methods are disclosed herein of using rapamycin compositions to treat or prevent diseases or conditions which a patient has been identified as being at risk for developing, including: feline chronic gingiva-stomatitis (FCGS) and other gum and gingival diseases, and other autoimmune diseases, all of which may occur in humans or other animals.

### PHARMACEUTICAL PREPARATIONS

Certain methods and compositions set forth herein are directed to administration of an effective amount of a composition comprising the rapamycin compositions of the present invention.

### 1. Compositions

A "pharmaceutically acceptable carrier": includes any and all solvents, dispersion media, coatings, surfactants, antioxidants, preservatives (e.g., antibacterial agents, antifungal agents), isotonic agents, absorption delaying agents, salts, preservatives, drugs, drug stabilizers, gels, binders, excipients, disintegration agents, lubricants, sweetening agents, flavoring agents, dyes, such like materials and combinations thereof, as would be known to one of ordinary skill in the art (Remington's, 1990). Except insofar as any conventional carrier is incompatible with the active ingredient, its use in the therapeutic or pharmaceutical compositions is contemplated. The compositions used in the present invention may comprise different types of carriers depending on whether it is to be administered in solid, liquid or aerosol form, and whether it needs to be sterile for such routes of administration as injection.

The use of such media and agents for pharmaceutically active substances is well known in the art. Except insofar as any conventional media or agent is incompatible with the active ingredient, its use in the therapeutic compositions is contemplated. Supplementary active ingredients can also be incorporated into the compositions, and these are discussed in greater detail below. For human administration, preparations should meet sterility, pyrogenicity, and general safety and purity standards as required by FDA Office of Biologics standards.

The formulation of the composition may vary depending upon the route of administration. For parenteral administration in an aqueous solution, for example, the solution should be suitably buffered if necessary and the liquid diluent first rendered isotonic with sufficient saline or glucose. In this connection, sterile aqueous media that can be employed will be known to those of skill in the art in light of the present disclosure.

In addition to the compounds formulated for parenteral administration, such as intravenous or intramuscular injection, other pharmaceutically acceptable forms include, e.g., tablets or solids for oral administration; liposomal and nanoparticle formulations; enteric coating formulations; time release capsules; formulations for administration via an implantable drug delivery device; and any other form. One may also use nasal solutions or sprays, aerosols or inhalants in the present invention.

The capsules may be, for example, hard-shell capsules or soft-shell capsules. The capsules may optionally include one or more additional components that provide for sustained release.

In certain embodiments, pharmaceutical composition includes at least about 0.1% by weight of the active compound. In other embodiments, the pharmaceutical composition includes about 2% to about 75% of the weight of the composition, or between about 25% to about 60% by weight of the composition, for example, and any range derivable therein.

The compositions may comprise various antioxidants to retard oxidation of one or more components. Additionally, the prevention of the action of microorganisms can be accomplished by preservatives such as various antibacterial and antifungal agents, including, but not limited to, parabens (e.g., methylparabens, propylparabens), chlorobutanol, phenol, scorbic acid, thimerosal or combinations thereof. The composition should be stable under the conditions of manufacture and storage, and preserved against the contaminating action of microorganisms, such as bacteria and fungi.

In certain preferred embodiments, an oral composition may comprise one or more binders, excipients, disintegration agents, lubricants, flavoring agents, and combinations thereof. When the dosage unit form is a capsule, it may contain, in addition to materials of the above type, carriers such as a liquid carrier. Various other materials may be present as coatings or to otherwise modify the physical form of the dosage unit. For instance, tablets, pills, or capsules may be coated with shellac, sugar, EUDRAGIT^{®} Acrylic Drug Delivery Polymers, or any combination thereof.

In particular embodiments, prolonged absorption can be brought about by the use in the compositions of agents delaying absorption, such as, for example, aluminum monostearate, gelatin, EUDRAGIT^{®} Acrylic Drug Delivery Polymers or combinations thereof.

### 2. Alternative Routes of Administration

Upon formulation, solutions will be administered in a manner compatible with the dosage formulation and in such amount as is therapeutically effective.

Absent clear limitation in a particular context, compositions according to these teachings can be administered to the subject using any method known to those of ordinary skill in the art. For example, a pharmaceutically effective amount of the composition may be administered intravenously, intracerebrally, intracranially, intraventricularly, intrathecally, into the cortex, thalamus, hypothalamus, hippocampus, basal ganglia, substantia nigra or the region of the substantia nigra, cerebellum, intradermally, intraarterially, intraperitoneally, intralesionally, anally, subcutaneously, orally, topically, locally, by inhalation (e.g., aerosol inhalation(, injection, infusion, continuous infusion, localized perfusion bathing target cells directly, via a catheter, via a lavage, in creams, in lipid compositions (e.g., liposomes), or by other method or any combination of the foregoing as would be known to one of ordinary skill in the art (Remington's, 1990).

In particular embodiments, the composition is administered to a subject using a drug delivery device. Any drug delivery device is contemplated for use in delivering an effective amount of the inhibitor of mTOR or mTOR Complex 1(mTORC1).

### 3. Dosage

A pharmaceutically effective amount of an inhibitor of mTOR is determined based on the intended goal. The quantity to be administered, both according to number of treatments and dose, depends on the subject to be treated, the state of the subject, the protection desired, and the route of administration. Precise amounts of the therapeutic agent also depend on the judgment of the practitioner and are peculiar to each individual.

The amount of rapamycin or rapamycin analog or derivative to be administered will depend upon the disease to be treated, the length of duration desired and the bioavailability profile of the implant, and site of administration. Generally, the effective amount will be within the discretion and wisdom of the patient's physician. Guidelines for administration include dose ranges of from about 0.01 mg to about 500 mg of rapamycin or rapamycin analog.

For example, a dose of the inhibitor of mTOR may be about 0.0001 milligrams to about 1.0 milligrams, or about 0.001 milligrams to about 0.1 milligrams, or about 0.1 milligrams to about 1.0 milligrams, or even about 10 milligrams per dose or so. Multiple doses can also be administered. In some embodiments, a dose is at least about 0.0001 milligrams. In further embodiments, a dose is at least about 0.001 milligrams. In still further embodiments, a dose is at least 0.01 milligrams. In still further embodiments, a dose is at least about 0.1 milligrams. In more particular embodiments, a dose may be at least 1.0 milligrams. In even more particular embodiments, a dose may be at least 10 milligrams. In further embodiments, a dose is at least 100 milligrams or higher.

In other non-limiting examples, a dose may also comprise from about 1 microgram/kg/body weight, about 5 microgram/kg/body weight, about 10 microgram/kg/body weight, about 50 microgram/kg/body weight, about 100 microgram/kg/body weight, about 200 microgram/kg/body weight, about 350 microgram/kg/body weight, about 500 microgram/kg/body weight, about 1 milligram/kg/body weight, about 5 milligram/kg/body weight, about 10 milligram/kg/body weight, about 50 milligram/kg/body weight, about 100 milligram/kg/body weight, about 200 milligram/kg/body weight, about 350 milligram/kg/body weight, about 500 milligram/kg/body weight, to about 1000 mg/kg/body weight or more per administration, and any range derivable therein. In non-limiting examples of a derivable range from the numbers listed herein, a range of about 5 mg/kg/body weight to about 100 mg/kg/body weight, about 5 microgram/kg/body weight to about 500 milligram/kg/body weight, *etc.,* can be administered, based on the numbers described above.

The dose can be repeated as needed as determined by those of ordinary skill in the art. Thus, in some embodiments of the methods set forth herein, a single dose is contemplated. In other embodiments, two or more doses are contemplated. In some embodiments, the two or more doses are the same dosage. In some embodiments, the two or more doses are different dosages. Where more than one dose is administered to a subject, the time interval between doses can be any time interval as determined by those of ordinary skill in the art. For example, the time interval between doses may be about 1 hour to about 2 hours; about 2 hours to about 6 hours; about 6 hours to about 10 hours; about 10 hours to about 24 hours; about 1 day to about 2 days; about 1 week to about 2 weeks, or longer, or any time interval derivable within any of these recited ranges. In specific embodiments, the composition may be administered daily, weekly, monthly, annually, or any range therein.

Doses for encapsulated rapamycin (enteric-coated rapamycin) and for encapsulated rapamycin nanoparticles may be different. According to preferred embodiments of the present invention, doses of the mTOR inhibitor are contemplated in a dosage range of between one microgram/kilogram and eight hundred micrograms/kilogram of the subject's weight. More particular dosage ranges for preferred embodiments are between about 50 micrograms and up to about 200 micrograms per kilogram for daily administration, or the equivalent for other frequencies of administration.

Although dosing may vary based on particular needs and preferred treatment protocols according to physician preference, maximum tolerable daily bioavailable dosings (trough levels) for a 28-day duration is preferably about 600 micrograms of rapamycin (or equivalent) per subject kilogram for feline subjects. As another example, preferred dosing for human subjects and canine subjects is at least about one microgram per kilogram of human weight and at least about ten micrograms per kilogram of canine weight. For both human and canine subjects, a more particular preferred maximum tolerable daily bioavailable dosing (trough levels) for a multi-week duration of four weeks or less is about two hundred micrograms of bioavailable rapamycin (or equivalent) per subject kilogram. Notwithstanding such examples, those of ordinary skill would understand that greater dose amount ranges may be tolerable and suitable when administered less often than once per day, and lesser ranges would be tolerable when administered more often than once per day.

In certain embodiments, it may be desirable to provide a continuous supply of a pharmaceutical composition to the patient. This could be accomplished by catheterization, followed by continuous administration of the therapeutic agent. The administration could be intra-operative or post-operative.

### 4. Secondary and Combination Treatments

Certain embodiments provide for the administration or application of one or more secondary or additional forms of therapies. The type of therapy is dependent upon the type of disease that is being treated or prevented. The secondary form of therapy may be administration of one or more secondary pharmacological agents that can be applied in the treatment or prevention of, for example, FCGS, gingivitis or other gingival disorder, or other autoimmune disorders or conditions associated with gingival disease or other autoimmune conditions in a patient who has been identified as being at risk for developing any of these conditions.

If the secondary or additional therapy is a pharmacological agent, it may be administered prior to, concurrently, or following administration of the inhibitor or mTOR.

The interval between administration of the inhibitor of mTOR and the secondary or additional therapy may be any interval as determined by those of ordinary skill in the art. For example, the inhibitor of mTOR and the secondary or additional therapy may be administered simultaneously, or the interval between treatments may be minutes to weeks. In embodiments where the agents are separately administered, one would generally ensure that a significant period of time did not expire between the time of each delivery, such that each therapeutic agent would still be able to exert an advantageously combined effect on the subject. For example, the interval between therapeutic agents may be about 12 hours to about 24 hours of each other and, more preferably, within about 6 hours to about 12 hours of each other. In some situations, it may be desirable to extend the time period for treatment significantly, however, where several days (2, 3, 4, 5, 6 or 7) to several weeks (1, 2, 3, 4, 5, 6, 7 or 8) lapse between the respective administrations. In some embodiments, the timing of administration of a secondary therapeutic agent is determined based on the response of the subject to the inhibitor of mTOR.

### 5. Kits

Kits are also contemplated as being used in certain aspects of the present invention. For instance, a rapamycin composition of the present invention can be included in a kit. A kit can include a container. Containers can include a bottle, a metal tube, a laminate tube, a plastic tube, a dispenser, a pressurized container, a barrier container, a package, a compartment, or other types of containers such as injection- or blow-molded plastic containers into which the hydrogels are retained. The kit can include indicia on its surface. The indicia, for example, can be a word, a phrase, an abbreviation, a picture, or a symbol.

Further, the rapamycin compositions of the present invention may also be sterile, and the kits containing such compositions can be used to preserve the sterility. The compositions may be sterilized via an aseptic manufacturing process or sterilized after packaging by methods known in the art.

### GENERAL USES OF THE ORAL MTOR PREPARATIONS

When orally administered daily, or at other regular frequencies, in correspondingly effective doses, pharmaceutical preparations prepared according to the foregoing descriptions, and their equivalents, are effective for preventing and treating various maladies in humans and other animals, and for reducing the progression of those maladies and their sequelae. For example, such oral administration enables a human or animal subject or the caregiver of such human or animal subject to prevent or treat various autoimmunities, and precursors, concomitants and sequelae thereof in humans and other animals.

Preferably, preparations according to the preferred embodiments are administered at a regular frequency, preferably at frequencies varying from three times per week (either on three consecutive days, or on three regularly distributed days of the week).

Although dosing may vary based on particular needs and preferred treatment protocols according to veterinarian preference, maximum tolerable daily bioavailable dosings (trough levels) for a 28-day duration are about 600 micrograms of rapamycin (or equivalent) per subject kilogram, for feline subjects. In contrast, preferred dosing for human subjects and canine subjects does not exceed preferred maximum tolerable daily bioavailable dosing (trough levels) for a multi-week duration of four weeks or less of about two hundred micrograms of bioavailable rapamycin (or equivalent) per subject kilogram, for both human and canine subjects. Nonetheless, those of ordinary skill would understand that greater dose amount ranges would be tolerable and suitable when administered less often than once per day, and lesser ranges would be tolerable when administered more often than once per day.

Whereas prior art uses of rapamycin may have involved recommended daily dosings of as low as roughly 13 micrograms per kilogram, FCGS protocols according to preferred embodiments of the present invention use higher dosings than the prior art, preferably in a range of more than 50 micrograms and up to (or even exceeding) 600 micrograms per kilogram for daily administration, or the equivalent for other frequencies of administration. Conditions addressed by oral administration protocols of the present invention include preventing and treating gingival diseases in humans, dogs and cats, whether through the preferred preparations of rapamycin (or the equivalent) or through combination therapies with stem cell therapy and/or other active pharmaceutical or botanical treatment protocols.

### SPECIFIC USES OF ORAL MTOR PREPARATIONS

The following disclosures describe uses of oral mTOR preparations for specific maladies, and the teachings of the present invention contemplate use of microencapsulated rapamycin nanoparticle preparations for these same purposes. In combination with background information regarding these maladies are specific example descriptions as observed by the inventors and their collaborators.

### Feline Chronic Gingivo-Stomatitis (FCGS), Gingivitis & Stomatitis in General

When orally administered daily, or at other regular frequencies (such as three times per week), in correspondingly effective doses, pharmaceutical preparations prepared according to the foregoing descriptions, and their equivalents, are thought to be effective for preventing and treating various autoimmune maladies in humans, canines, felines and other animals, and for delaying or reducing the progression of those maladies and their sequelae.

For example, when orally administered daily or three times per week, or at other regular frequencies, in correspondingly effective doses, pharmaceutical preparations prepared according to the foregoing descriptions, and their equivalents, are effective for preventing and treating and reducing the progression of various gingival diseases. Preferably, preparations according to the preferred embodiments are administered at a regular frequency, preferably in periods in excess of one year on a daily or three times per week regimen. Note that dosing may occur more frequently or less frequently. Particularly identified gingival diseases include gingivitis stomatitis (a/k/a gingivo-stomatitis), which includes diseases known as "lymphocytic" or "plasmacytic" gingivo-stomatitis.

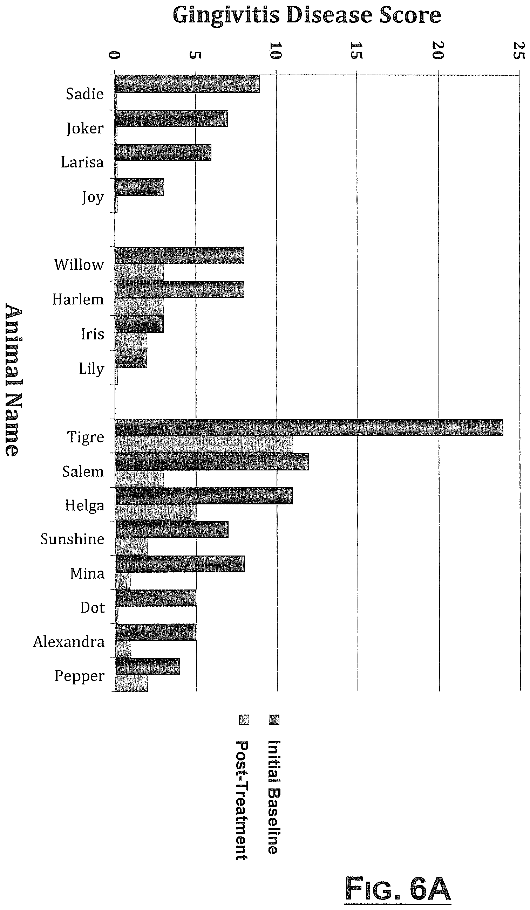

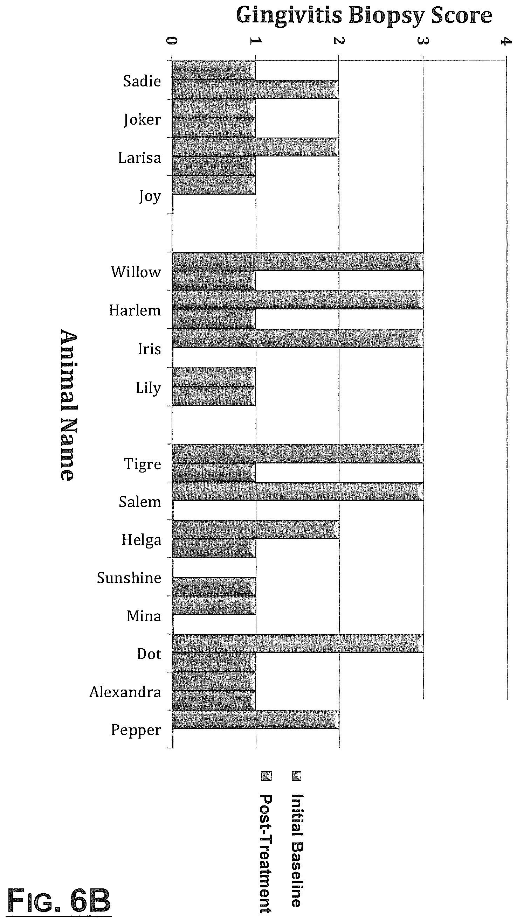

For instance, positive efficacy was observed in felines with chronic gingivo-stomatitis, when microencapsulated rapamycin nanoparticle preparations according to the present invention were administered three times a week orally, in capsules containing doses at 200, 400 and 600 micrograms/kilogram, variously for two-, four-, and six-week durations. Particularly, in controlled studies following a protocol that confirmed the initial presence of medium to severe FCGS, an autoimmune gingival disease, microencapsulated nanoparticle preparations produced according to the process illustrated in Figs. 2-4B not only stopped progression of FCGS in all subjects tested, but also significantly reduced the severity of FCGS in most if not all of the tested subjects. More particularly, as illustrated in **Fig.** 6 and discussed in more detail below in Examples 1 and 2, delineating the protocols of treating medium to severe FCGS with administration of microencapsulated nanoparticle preparations, produced according to the process illustrated in **Figs. 2-5B****,** three times a week over 6 consecutive weeks which showed reduced severity of FCGS in all of the feline subjects that participated in the studies.

Particularly beneficial results are appreciable through regular multi-week oral administration in the prevention and treatment of FCGS as well as other gingival diseases. "Regular" oral administration may include oral administration of capsules, tablets or other oral dosing forms of microencapsulated rapamycin nanoparticles (or their equivalents) at least twice weekly, and preferably at least three times weekly, while alternative treatment protocols may be achieved through multiple dosings per day as well.

In any particular treatment protocol, it should be appreciated that dosing may vary based on particular needs and preferred treatment protocols according to preference, and depending on weight, species and other characteristics of the particular subject as well as the particular stomatitis condition for which the protocol is being applied. Those of ordinary skill would understand that greater dose amount ranges would be tolerable and suitable when administered less often than once per day, and lesser ranges would be tolerable when administered more often than once per day.

### EXAMPLES

The following examples are included to demonstrate certain non-limiting aspects of the invention. It should be appreciated by those of skill in the art that the techniques disclosed in the example that follows represent techniques that are thought to function well in the practice of the invention. However, those of skill in the art, in light of the present disclosure, would also appreciate that many changes can be made in the specific embodiments that are disclosed and still obtain a like or similar result without departing from the scope of the claimed invention.

### EXAMPLE 1

Oral administration of microencapsulated rapamycin nanoparticles has been shown to be effective in reducing the severity of FCGS, with the results of one particular study protocol illustrated in the graphs of **Figs. 6A** and **6B** demonstrating such a reduction in severity.

Oral administration of eRapaNP2g in capsule form occurred over a 6-week period in order to evaluate the efficacy of the orally administered rapamycin in the treatment of feline gum disease. Animals were chosen based on oral examination revealing moderate persistent gingival inflammation and/or oral pathology representative of feline gingivo-stomatitis. Four days prior to the first administration of rapamycin, the study animals were examined and the affected tissues were assigned a score based, in part, on the severity of the condition according to pre-established study protocol, with these initial scores being plotted as the Baseline in **Fig. 6A****.** In assigning each study subject a score for baseline purposes, eight regions of the oral cavity were evaluated on examination and assigned a score on a 4-point scale based on severity of disease observed for each region. Specifically, the scale was characterized as follows: 0 - No inflammation; 1 - Mild inflammation, slight redness in color, slight edema, no bleeding on probing; 2 - Moderate inflammation, moderate glazing, redness, bleeding on probing. Mild proliferation or ulceration of the gums may be present; and 3 - Severe inflammation, marked redness and hypertrophy, ulceration, tendency to spontaneous bleeding, marked proliferation/ulceration of the gums. The eight regions of the oral cavity to which scores were assigned: upper canines and incisors; lower canines and incisors; upper right premolars and molars; upper left premolars and molars; lower right premolars and molars; lower left premolars and molars; right palatoglossal folds and fauces; and left palatoglossal folds and fauces. Therefore, the maximum total score for any study subject was 24 (8 regions x 3 maximum score per region). Overall, the mean baseline score across the study group was 7.6 out of a possible 24.

Biopsies of affected tissue were taken by punch biopsy or excision prior to eRapaNP2g administration at the initial oral examination and again following the oral examination at the termination of treatment, the tissues being submitted for histopathology analysis. The site of the biopsy was selected from the region of greatest inflammation in which a minimum of 3 mm biopsy punch could be collected. The final biopsy was taken from the region adjacent to the original biopsy site. Each study subject was assigned a baseline biopsy score rated on a 4-point scale corresponding to none, mild, moderate, or severe inflammation. These baseline biopsy scores are illustrated in the graph in **Fig. 6B****,** which also plots the post-treatment biopsy scores for each of the study subjects.

For the particular studies conducted by Applicant, each study subject was assigned a baseline biopsy score rated on a 4-point scale corresponding to none, mild, moderate, or severe inflammation. These baseline biopsy scores are illustrated in the graph in **Fig. 6B****,** which also plots the post-treatment biopsy scores for each of the study subjects.

Feline subjects were given between about 400 and 600 µg/kg of eRapaNP2g, a preferred form of e-nanoRapa. Within that range, specific desired dosages were resolved and determined based in part on the weight of each subject. For each treated subject, the same quantity of the same rapamycin preparation was administered on Days 1, 3, 5, 8, 10, and 12 during a first two-week regimen (Cycle 1). Then, after assessing results of the first two-week regimen, a desired quantity of the same rapamycin preparation was administered on Days 15, 17, 19, 22, 24, and 26 in a second 2-week regimen (Cycle 2) of the study, which immediately followed the first two-week regimen. For assessing results after the first two-week regimen, each subject animal had its oral cavity examined on day 13, following the end of Cycle 1. For assessing results after the second two-week regimen, each subject animal had its oral cavity examined on Day 27, following the end of Cycle 2.

Results of those treatments are illustrated as the "Post-Treatment Scores" for each study subject in **Fig. 6A****,** which show that in every instance the severity of gingival disease was reduced. Overall, the mean post-treatment score across the study group was 2.1 out of 24, down from a mean of 7.6 prior to administration of the first eRapaNP2g regimen. In all but one of the treated animals, the reduction of the severity of the disease was more than 50%. In 43.8% of the animals treated, the disease was nearly eliminated altogether. Differences in biopsy scores also indicate that eRapaNP2g is effective in treating FCGS. As illustrated in **Fig. 6B****,** the biopsy score for eleven out of the sixteen animals dropped after treatment with eRapaNP2g. Overall, the mean biopsy score for the study group dropped from 1.875 prior to drug administration to a mean of 0.75 after drug administration was completed. Thus, orally administered enteric-coated rapamycin or eRapaNP2g at a dosage of 400-600 µg/kg has shown to be effective in treating chronic gingivo-stomatitis in cats.

### EXAMPLE 2

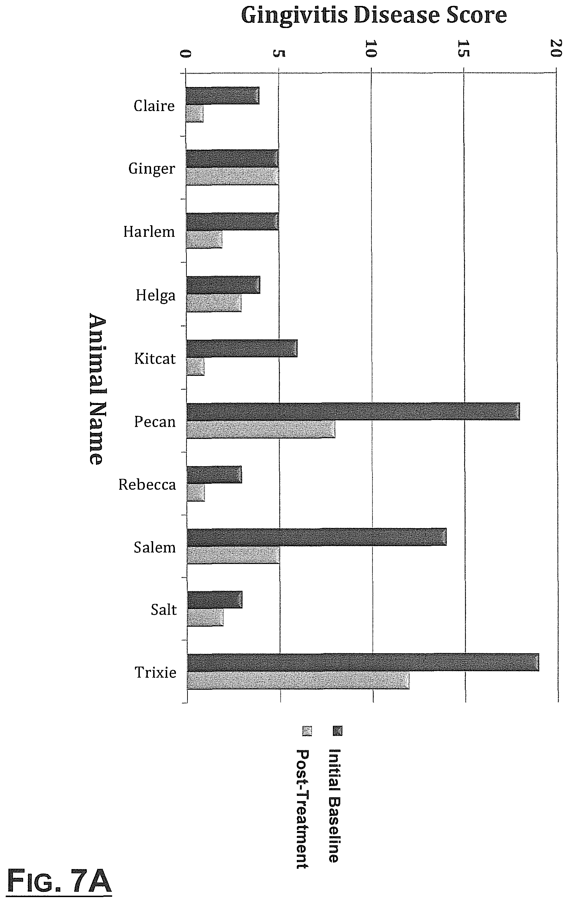

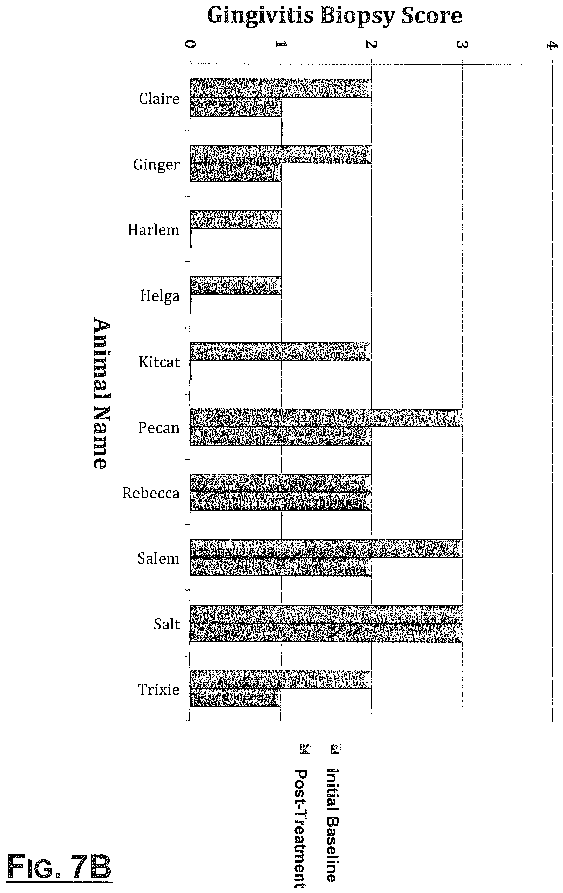

The goal of this study was to evaluate the effectiveness of rapamycin (eRapaNP2g) in treating chronic, persistent or refractory inflammatory diseases of the gums and oral cavity that are not responsive to dentistry and antibiotic therapy. Specifically, the present study examined the effectiveness of a lower dose of 200 µg/kg of eRapaNP2g. The results of the study are illustrated in **Figs. 7A** and **7B****.**

Thirteen domestic cats were selected to enter the trial based on inflammatory appearance of the gums at previous veterinary examinations. An additional criterion for selection was that of good general health (other than gingival inflammation) as determined by baseline veterinary physical examinations, urinalysis, and complete blood count and biochemistry analysis. The cats selected were of both sexes and ranged from 3 to 13 years of age. Baseline blood for a complete blood count (CBC) and superchemistry parameters and urine for urinalysis was collected from animals on Day -22. A full oral examination was carried out to determine the level of inflammatory gum disease. Following the oral examination, each study subject received a dental cleaning and 7 days of antibiotic therapy, with each animal receiving either a 62.5 mg dose or a 125 mg dose of Aventiclav (amoxicillin/clavulanic acid) twice daily, the dosage being based on the weight of the animal.

On Day -4, oral examination was repeated to determine the presence of any disease and each animal was then assigned a baseline score based on the severity of disease observed. Eight regions of the oral cavity (upper canines and incisors; lower canines and incisors; upper right premolars and molars; upper left premolars and molars; lower right premolars and molars; lower left premolars and molars; right palatoglossal folds and fauces; and left palatoglossal folds and fauces) were scored for each study subject on a 4-point scale. The scale was characterized as follows: 0 - No inflammation; 1 - Mild inflammation, slight redness in color, slight edema, no bleeding on probing; 2 - Moderate inflammation, moderate glazing, redness, bleeding on probing. Mild proliferation or ulceration of the gums may be present; 3 - Severe inflammation, marked redness and hypertrophy, ulceration, tendency to spontaneous bleeding, marked proliferation/ulceration of the gums. Since each of the eight regions was scored for each study subject, the maximum possible score for an individual subject was 24 (3 x 8). Blood and urine were also collected which provided baseline data. Three animals scored lower than 3 at the time of assessment, displaying a positive response to dental work and antibiotic therapy. These animals were not entered into the treatment phase of the trial. The animals that exhibited disease following the dentistry and antibiotic therapy on oral examination were selected for the treatment phase of the trial.

Biopsies were taken by punch biopsy or excision from the affected tissue and submitted for histopathology analysis after the initial oral examination. Baseline biopsies of gum tissue were collected, such biopsies being taken from the area of the gums which had the highest level of inflammation, and scores were assigned for each specimen based on a 4-point scale (0 - 3) corresponding to the level of inflammation observed rated as none, mild, moderate, or severe. The final biopsy was taken from the region adjacent to the original biopsy site. Baseline biopsy scores for each study subject, as well as post-treatment biopsy scores, are illustrated in **Fig. 6B****.**

The animals were given a three-day rest period to heal following the baseline assessment. Baseline Quantitative Magnetic Resonance (QMR) analysis was performed on all animals with this time period. The animals then entered two 2-week dosing cycles with eRapaNP2g. During the first 2-week drug administration cycle, eRapaNP2g was administered orally on Saturday, Monday and Wednesday (i.e. the drug was given every other day for 5 days). There was then a 2-day break, which was followed by the every other day treatment regime. One day after the last administered dose of the first 2-week dosing cycle, subjects underwent veterinary exams, after which blood and urine were collected and their gums examined and scored. At the end of the first dosing cycle, 8 out of 10 cases showed improvement based on gum scores. In the other two cases, there was no change. The second 2-week drug administration cycle resumed with the same dosing regimen described above (i.e. an every other day treatment schedule) with the same examination routine of each study subject occurring one day after the last administered dose in the cycle. There was little further improvement at the end of the second 2-week dosing cycle, with some animals showing partial reversion.

A third 2-week dosing cycle was added to the trial. At the completion of the third dosing cycle, final physical and oral examinations were performed and blood and urine were collected. In addition, biopsies were taken for subsequent histological analysis by the University of Guelph Animal Health Laboratory. As illustrated by the graph in **Fig. 6A****,** when compared to baseline, 9 out of 10 subjects showed improvement which was statistically significant (p=0.005). A second biopsy was also collected for submission to the bioanalytical laboratory for measurement of drug concentration with the gum tissue. According to the biopsy data, eight out of ten animals showed improvement and two animals were unchanged. Using the Wilcoxon Matched Pairs Test, the difference was found to be highly significant (t=2.5205; p=0.01117). The biopsy results are shown in **Fig. 6B****.** After completing the gum biopsies, QMR scans were performed on the last day of the study and compared with QMR results at baseline. There were no statistically significant changes, although there was a marginally significant increase in the ratio of fat to body weight (p=0.06).

Sixteen domestic cats were given 400-600 µg/kg of eRapaNP2g, and specific dosages were determined based on animal weight. Rapamycin was administered on Days 1, 3, 5, 8, 10, and 12 (Cycle 1), and 15, 17, 19, 22, 24, and 26 (Cycle 2) of the study. Each animal had its oral cavity examined on day 13, following the end of Cycle 1 and again on day 27, following the end of Cycle 2. Results of the treatments, illustrated as the post-treatment scores for each study subject in **Fig. 6A****,** show that in every instance the severity of the gingival disease was reduced. Overall, the mean post-treatment score across the study group was 2.1 out 24, down from a mean of 7.6 prior to administration of eRapaNP2g. In all but one of the treated animals, the reduction of the severity of the disease was more than 50%. In 43.8% of the animals treated, the disease was nearly eliminated altogether. Differences in biopsy scores also indicate that eRapaNP2g is effective in treating FCGS. As illustrated in **Fig. 6B****,** the biopsy score for eleven of the sixteen animals dropped after treatment with eRapaNP2g. Overall, the mean biopsy score for the study group dropped from 1.875 prior to drug administration to a mean of 0.75 after drug administration was completed. Thus, orally administered enteric-coated rapamycin or eRapaNP2g at a dosage of 400-600 µg/kg has shown to be effective in treating chronic gingivo-stomatitis in cats.

### EXAMPLE 2

The goal of this study was to evaluate the effectiveness of rapamycin (eRapaNP2g) in treating chronic, persistent or refractory inflammatory diseases of the gums and oral cavity that are not responsive to dentistry and antibiotic therapy. Specifically, the present study examined the effectiveness of a lower dose of 200 µg/kg of eRapaNP2g. The results of the study are illustrated in **Figs. 7A** and **7B****.**

Thirteen domestic cats were selected to enter the trial based on inflammatory appearance of the gums at previous veterinary examinations. An additional criterion for selection was that of good general health (other than gingival inflammation) as determined by baseline veterinary physical examinations, urinalysis, and complete blood count and biochemistry analysis. The cats selected were of both sexes and ranged from 3 to 13 years of age. Baseline blood for a complete blood count (CBC) and superchemistry parameters and urine for urinalysis was collected from animals on Day -22. A full oral examination was carried out to determine the level of inflammatory gum disease. Following the oral examination, each study subject received a dental cleaning and 7 days of antibiotic therapy, with each animal receiving either a 62.5 mg dose or a 125 mg dose of Aventiclav (amoxicillin/clavulanic acid) twice daily, the dosage being based on the weight of the animal.

On Day -4, oral examination was repeated to determine the presence of any disease and each animal was then assigned a baseline score based on the severity of disease observed. Eight regions of the oral cavity (upper canines and incisors; lower canines and incisors; upper right premolars and molars; upper left premolars and molars; lower right premolars and molars; lower left premolars and molars; right palatoglossal folds and fauces; and left palatoglossal folds and fauces) were scored for each study subject on a 4-point scale. The scale was characterized as follows: 0 - No inflammation; 1 - Mild inflammation, slight redness in color, slight edema, no bleeding on probing; 2 - Moderate inflammation, moderate glazing, redness, bleeding on probing. Mild proliferation or ulceration of the gums may be present; 3 - Severe inflammation, marked redness and hypertrophy, ulceration, tendency to spontaneous bleeding, marked proliferation/ulceration of the gums. Since each of the eight regions was scored for each study subject, the maximum possible score for an individual subject was 24 (3 x 8). Blood and urine were also collected which provided baseline data. Three animals scored lower than 3 at the time of assessment, displaying a positive response to dental work and antibiotic therapy. These animals were not entered into the treatment phase of the trial. The animals that exhibited disease following the dentistry and antibiotic therapy on oral examination were selected for the treatment phase of the trial.

Biopsies were taken by punch biopsy or excision from the affected tissue and submitted for histopathology analysis after the initial oral examination. Baseline biopsies of gum tissue were collected, such biopsies being taken from the area of the gums which had the highest level of inflammation, and scores were assigned for each specimen based on a 4-point scale (0, 1, 2 or 3) corresponding to the level of inflammation observed - "0" being rated as none, "1" being rated as mild, "2" being rated as moderate, and "3" being rated as severe. A final biopsy was taken from the region adjacent to the original biopsy site. Baseline biopsy scores for each study subject, as well as post-treatment biopsy scores, are illustrated in **Fig. 6B****.**

The animals were given a three-day rest period to heal following the baseline assessment. Baseline Quantitative Magnetic Resonance (QMR) analysis was performed on all animals with this time period. The animals then entered two 2-week dosing cycles with eRapaNP2g. During the first 2-week drug administration cycle, eRapaNP2g was administered orally on Saturday, Monday and Wednesday (i.e. the drug was given every other day for 5 days). There was then a 2-day break, which was followed by the every other day treatment regime. One day after the last administered dose of the first 2-week dosing cycle, subjects underwent veterinary exams, after which blood and urine were collected and their gums examined and scored. At the end of the first dosing cycle, 8 out of 10 cases showed improvement based on gum scores. In the other two cases, there was no change. The second 2-week drug administration cycle resumed with the same dosing regimen described above (i.e. an every other day treatment schedule) with the same examination routine of each study subject occurring one day after the last administered dose in the cycle. There was little further improvement at the end of the second 2-week dosing cycle, with some animals showing partial reversion.

A third 2-week dosing cycle was added to the trial. At the completion of the third dosing cycle, final physical and oral examinations were performed and blood and urine were collected. In addition, biopsies were taken for subsequent histological analysis by the University of Guelph Animal Health Laboratory. As illustrated by the graph in **Fig. 6A****,** when compared to baseline, 9 out of 10 subjects showed improvement which was statistically significant (p=0.005). A second biopsy was also collected for submission to the bioanalytical laboratory for measurement of drug concentration with the gum tissue. According to the biopsy data, eight out of ten animals showed improvement and two animals were unchanged. Using the Wilcoxon Matched Pairs Test, the difference was found to be highly significant (t=2.5205; p=0.01117). The biopsy results are shown in **Fig. 6B****.** After completing the gum biopsies, QMR scans were performed on the last day of the study and compared with QMR results at baseline. There were no statistically significant changes, although there was a marginally significant increase in the ratio of fat to body weight (p=0.06).

### ALTERNATIVE EMBODIMENTS WITH OTHER RAPAMYCINS

Although many aspects of the present invention relate directly to rapamycin itself, possible broader aspects of the invention relate also to analogs and derivatives of rapamycin, and to producing a more stable and effective oral preparation for delivering an agent to bind, interact with or otherwise regulate activity of the mTOR pathway.

Accordingly, as alternatives that benefit from many but not necessarily all of the teachings of the present invention, any of the particular embodiments described above may be modified by substituting one or more other rapamycins in place of (or in addition to) rapamycin. For corresponding purposes of these descriptions, rapalogs and all mTOR pathway inhibitors should be considered as "rapamycins" (i.e., the plural of rapamycin). Also, in this context and wherever else a context relates to any of the rapamycins rather than just rapamycin, any related references to "encapsulated rapamycin" should be read as teaching not only about discrete particles that include rapamycin, but also about discrete particles that include any one or more rapamycins. It should also be understood that reference to any "encapsulated" form (including "microencapsulated" forms) should be interpreted to disclose a form that in some embodiments is fully encapsulated; provided however, that reference to an "encapsulated" form does not necessarily mean that all embodiments of that form are completely encapsulated; rather, it should be understood that reference to an "encapsulated" form, without more, encompasses a form that may only be partially encapsulated, except to the extent clarified or reasonably understood otherwise.

### ADMINISTRATION IN COMBINATION WITH OTHER THERAPIES

It should also be understood that the foregoing disclosure emphasizes certain specific embodiments of the invention. Alternative embodiments may involve administration of rapamycin in combination with other therapies. Such therapies include but are not limited to dental scaling; long term use of antibacterial dental hygiene products; professional scaling and long-term tooth brushing with 0.2% chlorohexidine; corticosteroids; gold salts; antibiotics; chlorohexidine gluconate gel; radical dental extraction techniques of premolars, molars or other teeth; radiation therapy; cryotherapy; antibiotics with activity against gram-negative and anaerobic organisms (including amoxicillin-clavulanic acid combination, enrofloxacin, lincomycin, clindamycin, spiramycin, metrodinazole, and tetracyclines); subgingival injection of up 10 milligrams triamcinolone; long-term prednisolone, methylprednisolone, or triamcinolone; methylpredinisolone; sodium aurothimalate; aurothioglucose; azathioprine; cyclophosphamide; chlorambucil; immunostimulatory; PIND-ORF; megoestrol acetate; lactoferrin; sodium salicylate; meloxicam; interferon; thalidomide; anti-viral agents; AZT; PMEA; soft-tissue lasers; multivitamin; antioxidant supplementation; and chemical cautery.

### GENERAL ALTERNATIVES

In all respects, it should also be understood that the drawings and detailed description herein are to be regarded in an illustrative rather than a restrictive manner, and are not intended to limit the invention to the particular forms and examples disclosed. Rather, the invention includes all embodiments and methods within the scope of the invention as claimed.

It should be understood that any limitation discussed with respect to one embodiment of the invention may apply to any other embodiment of the invention. Moreover, any composition of the invention may be used in any method of the invention, and any method of the invention may be used to produce or to utilize any composition of the invention. Any embodiment of the present invention may consist of or consist essentially of - rather than comprise/include/ contain/have - the described features and/or steps.

## Claims

1. A microparticle pharmaceutical preparation for use in treating feline chronic gingivo-stomatitis (FCGS) through oral administration of said microparticle pharmaceutical preparation, comprising:
i. a solid excipient matrix comprising a polymer composition that remains intact when exposed to acidic conditions of the alimentary canal of a typical domestic feline subject and that disintegrates in basic conditions of intestinal portions of said alimentary canal; and
ii. mTOR inhibiting nanoparticles dispersed within said solid excipient matrix, said mTOR inhibiting nanoparticles comprising pharmaceutically active cores;
iii. said pharmaceutically active cores comprising a pharmaceutically active mTOR inhibiting compound;
iv. said mTOR inhibiting nanoparticles further comprising an aqueous-soluble, amphoteric compound;
v. said amphoteric, aqueous-soluble compound comprising sodium cholate;
vi. orally administering said microparticle pharmaceutical preparation to a feline subject having FCGS; and
vii. repeating oral administration of said microparticle pharmaceutical preparation to said feline subject multiple times per week over a multi-week duration, in amounts such that said feline subject ingests said pharmaceutically active compound in amounts and over durations that are efficacious for reducing the severity of FCGS in said feline subject.

2. The microparticle pharmaceutical preparation for use of claim 1, wherein said orally administering step comprises orally administering said microparticle pharmaceutical preparation at a frequency of three or more times per week, in a dosage that is therapeutically effective for preventing or treating autoimmune maladies in said feline subject when administered at said frequency.

3. The microparticle pharmaceutical preparation for use of claim 1, wherein said repeating oral administration step comprises administering said microparticle pharmaceutical preparation in amounts such that said feline subject ingests said compound in amounts, frequencies and over durations that are efficacious for treating as one or more of the following: gingival disease; Feline Chronic Gingivo-Stomatitis; autoimmune mucous membrane oropharyngeal maladies; and precursors, concomitants and sequelae of any of the foregoing.

4. The microparticle pharmaceutical preparation for use of claim 1, wherein said microparticle pharmaceutical preparation is administered to said feline subject wherein said feline subject is in need of prevention or treatment, or delayed progression, of feline chronic gingivo-stomatitis.

5. The microparticle pharmaceutical preparation for use of claim 4, wherein a dosage of said microparticle pharmaceutical preparation provides between 50 micrograms/kilogram and 800 micrograms/kilogram weight of said feline subject.

6. The microparticle pharmaceutical preparation for use of claim 5, wherein said dosage is administered at least three times per week for a duration of two, four, six or eight weeks.

7. The microparticle pharmaceutical preparation for use of claim 1, wherein said mTOR inhibiting nanoparticles comprise micelles.

8. The microparticle pharmaceutical preparation for use of claim 1, wherein said amphoteric, aqueous-soluble compound has properties that tend to naturally induce the formation of micelles within an aqueous solution of said amphoteric, aqueous-soluble compound.

9. The microparticle pharmaceutical preparation for use of claim 7, wherein said mTOR inhibiting nanoparticles have properties that promote stability when said mTOR inhibiting nanoparticles are dispersed within said solid excipient matrix.

10. The microparticle pharmaceutical preparation for use of claim 1, wherein said polymer composition is a methyl-methacrylate polymer composition.

## Patentansprüche

1. Pharmazeutische Mikropartikelformulierung zur Verwendung bei der Behandlung von feliner chronischer Gingivo-Stomatitis (FCGS) durch orale Verabreichung der pharmazeutischen Mikropartikelformulierung, umfassend:
i. eine feste Hilfsstoffmatrix, die eine Polymerzusammensetzung umfasst, die intakt bleibt, wenn sie den sauren Bedingungen des Verdauungskanals eines typischen domestischen felinen Patienten ausgesetzt ist, und die bei basischen Bedingungen der Darmabschnitte des Verdauungskanals zerfällt; und
ii. mTOR-hemmende Nanopartikel, die innerhalb der festen Hilfsstoffmatrix dispergiert sind, wobei die mTOR-hemmenden Nanopartikel pharmazeutisch aktive Kerne umfassen;
iii. die pharmazeutisch aktiven Kerne, die eine pharmazeutisch aktive mTOR-hemmende Verbindung umfassen;
iv. die mTOR-hemmenden Nanopartikel, die ferner eine wasserlösliche, amphotere Verbindung umfassen;
v. die amphotere, wasserlösliche Verbindung, die Natriumcholat umfasst;
vi. orales Verabreichen der pharmazeutischen Mikropartikelformulierung an einen felinen Patienten mit FCGS; und
vii. Wiederholen der oralen Verabreichung der pharmazeutischen Mikropartikelformulierung an den felinen Patienten mehrmals pro Woche über einen mehrwöchigen Zeitraum, in solchen Mengen, dass der feline Patient die pharmazeutisch aktive Verbindung in Mengen und über Dauern einnimmt, die bei der Reduzierung des Schweregrads von FCGS bei dem felinen Patienten wirksam sind.

2. Pharmazeutische Mikropartikelformulierung zur Verwendung nach Anspruch 1, wobei der orale Verabreichungsschritt orales Verabreichen der pharmazeutischen Mikropartikelformulierung mit einer Häufigkeit von drei oder mehr Malen pro Woche in einer Dosierung umfasst, die therapeutisch wirksam ist, um Autoimmunerkrankungen bei dem felinen Patienten bei dieser Verabreichungshäufigkeit vorzubeugen oder zu behandeln.

3. Pharmazeutische Mikropartikelformulierung zur Verwendung nach Anspruch 1, wobei das Wiederholen des oralen Verabreichungsschritts das Verabreichen der pharmazeutischen Mikropartikelformulierung in solchen Mengen umfasst, dass der feline Patient die Verbindung in Mengen, Häufigkeiten und über Dauern einnimmt, die bei der Behandlung einer oder mehrerer der folgenden wirksam sind: Zahnfleischerkrankung; feliner chronischer Gingivo-Stomatitis; Autoimmunerkrankungen der oropharyngealen Schleimhaut; und Vorläufer-, Begleiterscheinungen und Folgeerscheinungen beliebiger der vorgenannten.

4. Pharmazeutische Mikropartikelformulierung zur Verwendung nach Anspruch 1, wobei die pharmazeutische Mikropartikelformulierung dem felinen Patienten verabreicht wird, wobei der feline Patient einer Vorbeugung oder Behandlung oder verzögerten Progression der felinen chronischen Gingivo-Stomatitis bedarf.

5. Pharmazeutische Mikropartikelformulierung zur Verwendung nach Anspruch 4, wobei eine Dosierung der pharmazeutischen Mikropartikelformulierung zwischen 50 Mikrogramm/Kilogramm und 800 Mikrogramm/Kilogramm Gewicht des felinen Patienten bereitstellt.

6. Pharmazeutische Mikropartikelformulierung zur Verwendung nach Anspruch 5, wobei die Dosierung mindestens dreimal pro Woche über eine Dauer von zwei, vier, sechs oder acht Wochen verabreicht wird.

7. Pharmazeutische Mikropartikelformulierung zur Verwendung nach Anspruch 1, wobei die mTOR-hemmenden Nanopartikel Mizellen umfassen.

8. Pharmazeutische Mikropartikelformulierung zur Verwendung nach Anspruch 1, wobei die amphotere, wasserlösliche Verbindung Eigenschaften aufweist, die dazu neigen, die Bildung von Mizellen innerhalb einer wässrigen Lösung der amphoteren, wasserlöslichen Verbindung auf natürliche Weise zu induzieren.

9. Pharmazeutische Mikropartikelformulierung zur Verwendung nach Anspruch 7, wobei die mTOR-hemmenden Nanopartikel Eigenschaften aufweisen, die Stabilität fördern, wenn die mTOR-hemmenden Nanopartikel innerhalb der festen Hilfsstoffmatrix dispergiert sind.

10. Pharmazeutische Mikropartikelformulierung zur Verwendung nach Anspruch 1, wobei die Polymerzusammensetzung eine Methyl-Methacrylat-Polymerzusammensetzung ist.

## Revendications

1. Préparation pharmaceutique microparticulaire destinée à être utilisée dans le traitement d'une gingivo-stomatite chronique féline (FCGS) par administration orale de ladite préparation pharmaceutique microparticulaire, comprenant :
i. une matrice excipiente solide comprenant une composition polymère qui reste intacte lorsqu'elle est exposée à des conditions acides du tube digestif d'un sujet félin domestique type et qui se désintègre dans des conditions basiques de parties intestinales dudit tube digestif ; et
ii. des nanoparticules inhibant mTOR, dispersées dans ladite matrice excipiente solide, lesdites nanoparticules inhibant mTOR comprenant des noyaux pharmaceutiquement actifs ;
iii. lesdits noyaux pharmaceutiquement actifs comprenant un composant inhibant mTOR pharmaceutiquement actif ;
iv. lesdites nanoparticules inhibant mTOR comprenant en outre un composé amphotère soluble dans l'eau ;
v. ledit composé amphotère soluble dans l'eau comprenant du cholate de sodium ;
vi. l'administration orale de ladite préparation pharmaceutique microparticulaire à un sujet félin atteint de FCGS ; et
vii. la répétition de l'administration orale de ladite préparation pharmaceutique microparticulaire audit sujet félin plusieurs fois par semaine pendant une durée de plusieurs semaines, en quantités telles que ledit sujet félin ingère ledit composé pharmaceutiquement actif en quantités et sur des durées qui sont efficaces pour réduire la gravité de la FCGS chez ledit sujet félin.

2. Préparation pharmaceutique microparticulaire destinée à être utilisée selon la revendication 1, dans laquelle ladite étape d'administration orale consiste à administrer oralement ladite préparation pharmaceutique microparticulaire à une fréquence de trois fois ou plus par semaine, selon une posologie qui est thérapeutiquement efficace pour prévenir ou traiter des maladies auto-immunes chez ledit sujet félin lorsque l'administration a lieu à ladite fréquence.

3. Préparation pharmaceutique microparticulaire destinée à être utilisée selon la revendication 1, dans laquelle ladite étape de répétition de l'administration orale consiste à administrer ladite préparation pharmaceutique microparticulaire en quantités telles que ledit sujet félin ingère ledit composé en quantités et en fréquences et sur des durées qui sont efficaces pour traiter une ou plusieurs des maladies suivantes : maladie gingivale ; gingivo-stomatite chronique féline ; maladies auto-immunes bucco-pharyngées de la muqueuse ; et symptômes précurseurs, symptômes simultanés et séquelles de l'une quelconque des maladies précédentes.

4. Préparation pharmaceutique microparticulaire destinée à être utilisée selon la revendication 1, ladite préparation pharmaceutique microparticulaire étant administrée audit sujet félin, ledit sujet félin ayant besoin d'une prévention ou d'un traitement ou d'une progression retardée de la gingivo-stomatite chronique féline.

5. Préparation pharmaceutique microparticulaire destinée à être utilisée selon la revendication 4, une posologie de ladite préparation pharmaceutique microparticulaire fournissant entre 50 microgrammes/kilogramme et 800 microgrammes/kilogramme en poids dudit sujet félin.

6. Préparation pharmaceutique microparticulaire destinée à être utilisée selon la revendication 5, dans laquelle ladite posologie est administrée au moins trois fois par semaine pendant une durée de deux, quatre, six ou huit semaines.

7. Préparation pharmaceutique microparticulaire destinée à être utilisée selon la revendication 1, dans laquelle lesdites nanoparticules inhibant mTOR comprennent des micelles.

8. Préparation pharmaceutique microparticulaire destinée à être utilisée selon la revendication 1, dans laquelle ledit composé amphotère soluble dans l'eau possède des propriétés qui ont tendance à induire naturellement la formation de micelles dans une solution aqueuse dudit composé amphotère soluble dans l'eau.

9. Préparation pharmaceutique microparticulaire destinée à être utilisée selon la revendication 7, dans laquelle lesdites nanoparticules inhibant mTOR possèdent des propriétés qui favorisent une stabilité quand lesdites nanoparticules inhibant mTOR sont dispersées dans ladite matrice excipiente solide.

10. Préparation pharmaceutique microparticulaire destinée à être utilisée selon la revendication 1, dans laquelle ladite composition polymère est une composition polymère de méthacrylate de méthyle.
